# EUROPEAN PATENT APPLICATION

(11) **EP 4 574 165 A1**
(43) Date of publication of application: **25.06.2025**
(21) Application number: 23307335.2
(22) Date of filing: 21.12.2023
(51) Int. Cl.: A61K 38/20, C07K 14/55, C07K 14/705

(54) **IMMUNOCYTOKINE FOR CANCER TREATMENT**

(71) Applicant: Egle Therapeutics, 75014 Paris (FR)
(72) Inventor: Kotsias, Fiorella, Suresnes (FR); Tkach, Mercedes, Suresnes (FR); Zucchetti, Andres, Suresnes (FR); Vanhove, Bernard, Suresnes (FR); Piaggio, Eliane, Cedex 05 Paris (FR); Sedlik, Christine, Cedex 05 Paris (FR); Borcoman, Edith, Cedex 05 Paris (FR); Guardado Calvo, Pablo, Paris (FR); Caudana, Pamela, Cambridge (US)
(74) Representative: Ipsilon NNY

(57) **Abstract**

The invention relates to immunocytokines comprising an anti-CTLA-4 antibody fused to a Treg-specific IL-2 receptor antagonist and their use to selectively deplete tumor-associated Tregs, in particular for treating cancer.

## Description

### FIELD OF THE INVENTION

The invention pertains to the field of immunotherapy. The invention relates to immunocytokines comprising an anti-CTLA-4 antibody fused to a regulatory T cell (Treg)-specific IL-2 receptor antagonist and their use to selectively deplete tumor-associated Tregs, in particular for treating cancer.

### BACKGROUND OF THE INVENTION

Regulatory T cells (Tregs) are a key component of the microenvironment of several tumour types that prevent effector T cells and NK cells from mediating an immune response. Eliminating Treg cells from the tumour microenvironment restores immune responses and contribute to the elimination of the tumour (Exemplified by Curiel et al., Nat. Med., 2004, 10, 942-949; Hiraoka et al., Clin. Cancer Res., 2006, 12, 5423-5434; Stirm et al, Journal for ImmunoTherapy of Cancer 2023;11:e006263; and reviewed by Shan et al, Trends in Cancer, November 2022, Vol. 8, No. 11 https://doi.org/10.1016/j.trecan.2022.06.008). Given the role for Tregs in protection against autoimmune diseases, immunointervention aiming at depleting Treg cells must selectively deplete only tumor infiltrating Tregs (Fontenot et al., Nat. Immunol., 2003, 4, 330-336).

Treg cell survival is under the dependency of interleukin-2 (IL-2), which is mainly produced by effector T cells and NK cells, and signals in Treg cells via the high affinity IL-2 receptor (IL-2R) complex composed of alpha (IL-2-RA or CD25), beta (IL-2RB or CD122) and gamma (IL-2RG or Gamma c or CD132) chains. The alpha chain binds IL-2 with low affinity and does not participate in signaling. The combination of the beta and gamma chains forms an intermediate affinity receptor expressed on the effector T cells (conventional T (Tconv) cells (CD4+Foxp3-), CD8+) and NK cells responsible for the cell-mediated immune responses; and all three receptor chains form a high affinity receptor expressed constitutively on regulatory T cells (CD4+Foxp3+) which have immunosuppressive function, and shortly or transiently by activated effector T cells and NK cells.

Various IL-2 variants (IL-2 muteins) have been generated to modulate IL-2 biological activity for cancer treatment (Review in Leon et al., Seminars in Oncology, 2018, 45, 95-104). The no-alpha mutein is an agonist of IL-2R signaling with a reduced ability to expand Treg in vivo (Carmenate et al., J. Immul., 2013, 190, 6230-6238 ; Rojas et al., J. Mol. Recognit., 2015, 25, 261-268). The plus-beta mutein or IL-2 superkine is an agonist of IL-2R signaling with preferential in vivo expansion of CD8+ T cells and NK cells, with some effects on Tregs (Levin et al., Nature, 2012, 484, 529-533). The no-gamma mutein is an antagonist of IL-2R signaling with some preferential affinity for Tregs (Carmenate et al., J. Immunol., 2018, 200, 3475-3484). In mice, the no-alpha and plus-beta muteins have higher antitumor activity and lower toxicity than wild-type IL-2. The no-gamma mutein has antitumoral effect similar to anti-CD25 monoclonal antibodies in mice. Other antagonists of IL-2R signaling having the ability to inhibit Treg division by depriving them from wild-type IL-2 signaling while leaving active IL-2 signaling in CD8+ T cells are disclosed in WO 2020/201095 (IL-2V1; IL2-V4; IL-2V5; IL-2V6).

Anti-tumor immune responses are also under the control of various immune check-point proteins (PD-1, PD-L1, CTLA-4, LAG-3) that have been targeted with blocking monoclonal antibodies (immune checkpoint inhibitors) now used alone or in combination to treat some types of cancer. CTLA-4 or CTLA4 (cytotoxic T-lymphocyte-associated protein 4), also known as CD152, is a protein receptor that functions as an immune checkpoint and downregulates immune responses. CTLA-4 is constitutively expressed in regulatory T cells but only upregulated in conventional T cells after activation. It acts as an "off" switch when bound to CD80 and CD86 on the surface of antigen-presenting cells. Various CTLA-4 inhibitors such as Ipilimumab and tremelimumab (both FDA approved) have been developed for the treatment of various cancers such as metastatic melanoma, malignant pleural mesothelioma, metastatic non-small cell lung cancer (NSCLC), prostate cancer, renal cell carcinoma and hepatocellular carcinoma (Review in Oncology Live, June 22, 2023, 24, 11, 39-). Although a percentage of patients respond to these therapies, either as single agent or in combination, the high incidence of adverse events limits their efficacy.

To improve cancer treatment, there is a need for therapies aiming at selectively depleting tumor infiltrating Tregs.

### SUMMARY OF THE INVENTION

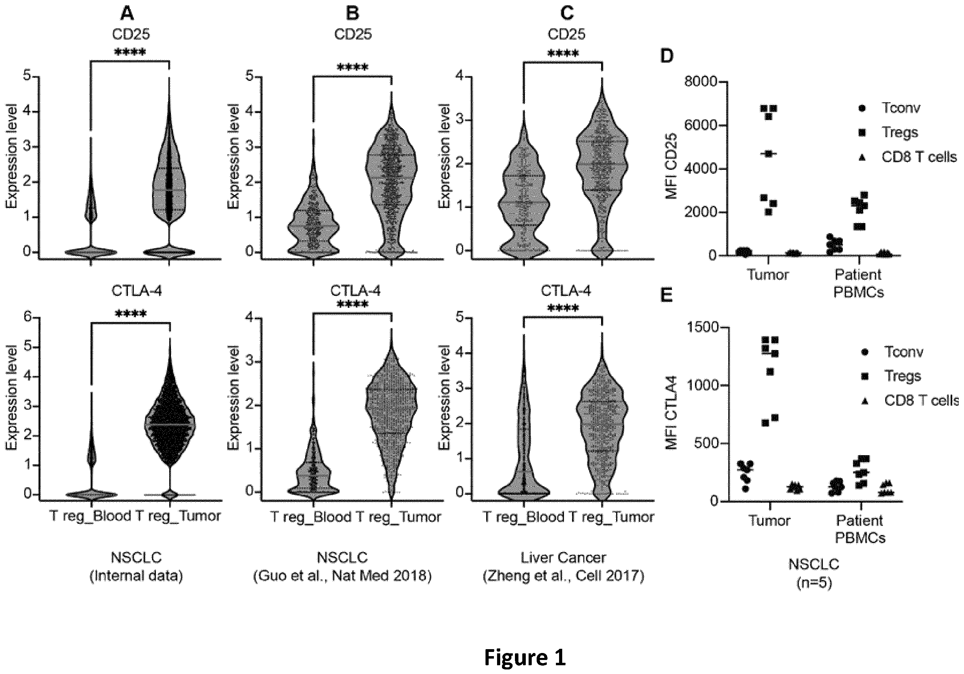

Using single-cell RNA sequencing data from cancer patients, and verified by FACS at the protein level, the inventors have identified that the interleukin-2 receptor alpha chain (IL2-RA or CD25) and cytotoxic T-lymphocyte associated protein 4 (CTLA-4, also known as CD152) are selectively overexpressed in tumor-associated Tregs as compared with peripheral Tregs or other T cell populations (Tconv and CD8 T cells) from tumors or blood (**Figure 1**)**.**

Based on these findings, the inventors have developed a novel immunocytokine (ICK) targeting the CTLA-4 and CD25 membrane proteins preferentially expressed on tumor-associated Tregs. This immunocytokine is combining the therapeutic modalities of an anti-CTLA-4 checkpoint inhibitor such as ipilimumab (humanized IgG1) and of an IL-2 mutein selectively targeting CD25-expressing cells previously disclosed in WO 2020/201095.

The inventors have shown that the immunocytokine is able to induce CD25 endocytosis, a property which is absent from the previously disclosed IL-2 muteins and provides an additional mechanism to interfere with CD25-mediated survival signals in Tregs. As a result, the immunocytokine is able to kill Tregs selectively and with high efficiency compared to anti-CTLA-4 antibody or IL-2 mutein used alone or in combination. Ex-vivo experiment confirms that the immunocytokine can selectively deplete Treg cells in a representative human tumor microenvironment. This specific combination of antibody and IL-2 mutein in an immunocytokine thus provides key properties to eliminate tumor-associated Tregs and promote an efficient anti-tumor response. Among, the immunocytokines tested, immunocytokine derived from one of the IL2 muteins (IL-2V5) also showed superior developability performance (higher production titers, higher purity yields and higher stability), combined with a lower immunogenicity (in silico) and best pharmacokinetic parameters (in mice and in vitro) as compared to immunocytokines derived from other IL2 muteins (IL-2V1 and IL-2V6). In view of these results, this novel immunocytokine represents a particularly promising candidate for the development of a new drug for cancer treatment.

Therefore, the invention relates to a fusion protein (immunocytokine) comprising an anti-CTLA-4 antibody, and an IL-2 variant which is a Treg-specific IL-2 receptor antagonist comprising the substitutions K9E, L12E, H16R, L19R, M23L, N26K, S87N, V91K, E95K, N119K, T123A; and further comprising the subsitutions S127K; Y31P and S127K; or K49Q, E52S, R81E, D84N, T131R and L132S, the indicated positions being determined by alignment with human IL-2 (SEQ ID NO: 1), and wherein the N-terminus of the IL-2 variant is fused to the C-terminus of the antibody heavy chain.

The immunocytokine of the invention induces CD25 endocytosis.

In some embodiments, the immunocytokine of the invention induces Treg apoptosis.

In some embodiments, the immunocytokine of the invention, depletes selectively tumor-associated Tregs.

In some embodiments, the anti-CTLA-4 antibody is ipilimumab, tremelimumab, or a functional variant thereof.

In some particular embodiments, the anti-CTLA-4 antibody comprises a H-CDR1 of SEQ ID NO: 2, H-CDR2 of SEQ ID NO: 3, a H-CDR3 of SEQ ID NO: 4, a L-CDR1 of SEQ ID NO: 5, a L-CDR2 of SEQ ID NO: 6 and a L-CDR3 of SEQ ID NO: 7 or variants thereof further having one or more conservative substitutions on one or more of these CDRs. In some more particular embodiments, the anti-CTLA-4 antibody comprises a VH domain having at least 85 % identity with SEQ ID NO: 8 and a VL domain having at least 85 % identity with SEQ ID NO:9.

In some embodiments, the anti-CTLA-4 antibody comprises a human IgG1 Fc domain and/or a human Ig kappa light chain constant domain, or is a nanobody.

In some embodiments, the IL-2 variant comprises an amino acid sequence having at least 85 % identity with any one of SEQ ID NO: 13 to 15.

In some embodiments, the immunocytokine according to the invention further comprises a linker between the antibody heavy chain C-terminus and IL-2 variant N-terminus; preferably comprising the sequence SEQ ID NO: 10.

In some particular embodiments, the fusion protein according to the invention comprises:
- an anti-CTLA-4 antibody heavy chain fused to the IL-2 variant, wherein said heavy chain fusion has at least 85%, 90%, 95% or 98% sequence identity with SEQ ID NO: 11; and
- an anti-CTLA-4 antibody light chain having at least 85%, 90%, 95% or 98% sequence identity with SEQ ID NO: 12.

Another aspect of the invention relates to a pharmaceutical composition comprising a therapeutically effective amount of the fusion protein according to the present disclosure. The invention also relates to the pharmaceutical composition according to the present disclosure for use in the treatment of cancer. In some embodiments, the cancer is selected from the group consisting of: Melanoma, Renal Cell Carcinoma, Colorectal Cancer, Hepatocellular Carcinoma, Non-Small Cell Lung Cancer, Malignant Pleural Mesothelioma, Esophageal Cancer, Head and Neck Squamous Cell Carcinoma, Urothelial Carcinoma, Primary Hodgkin Lynphoma, Gastric Cancer, Large B Cell Lymphoma, Cervical Cancer, Merkel Cell Carcinoma, Endometrial Carcinoma, Cutaneous Squamous Cell Carcinoma, Triple Negative Breast Cancer, Breast invasive Carcinoma, Pancreatic Adenocarcinoma, Thymoma, Prostate Adenocarcinoma, Ovarian Serous Cystadenocarcinoma, Thyroid Carcinoma, and Sarcoma. In some embodiments, the pharmaceutical composition is used in combination with at least one immune check-point inhibitor; preferably anti-PD-1 and/or anti-PDL-1.

### DETAILED DESCRIPTION OF THE INVENTION

The invention relates to immunocytokines comprising an anti-CTLA-4 antibody fused to an IL-2 mutein with IL-2 receptor antagonist activity specific for Tregs and their use to selectively deplete tumor-associated regulatory T cells, in particular for treating cancer.

### Definitions

As used herein "regulatory T cells", "Treg" or "Tregs" refer to CD3+CD4+Foxp3+ T cells, including CD3+CD4+Foxp3+CD25+ and CD3+CD4+Foxp3+CD25- cells.

As used herein, "effector cells" refer to effector T cells and NK cells. Effector T cells (Teff or Teffs) refer to one or more of conventional T (Tconv) cells and CD8+ T cells. Tconv are CD3+CD4+Foxp3- cells. CD8+ T cells are CD3+CD8+ cells. NK cells are CD3-CD16+ cells.

As used herein, "tumor-associated Treg" refers to activated Treg among tumor-infiltrating Tregs. In some particular embodiments, "tumor-associated Treg" refers to a distinct and isolated population (or group, subset or cluster) of CD4+ Foxp3+ cells that distinguishes from the heterogeneous pool of Tregs in that: (i) it is increased in the tumor, and eventually also in the tumor draining-lymph node(s); (ii) it is enriched with clonally expanded TCR specificities in the diseased-tissue; and (iii) it is enriched with a transcriptomic signature of T cell Receptor (TCR) triggering, cell activation and expansion, as disclosed in WO 2021/165546.

The term "antibody" herein is used in the broadest sense and includes polyclonal and monoclonal antibodies, including intact antibodies and functional (antigen-binding) antibody fragments, including fragment antigen binding (Fab) fragments; Fab', Fab'-SH, F(ab')2 fragments; Fv fragments; recombinant IgG (rIgG) fragments; variable heavy chain (VH) regions capable of specifically binding the antigen; single chain antibody fragments, including single chain variable fragments (scFv), and single domain antibodies (e.g., sdAb, sdFv, nanobody) fragments. The term "antibody" encompasses any CDR3 containing moiety. The term encompasses recombinant and/or otherwise modified forms of immunoglobulins, such as intrabodies, peptibodies, chimeric antibodies, fully human antibodies, heavy chain only antibodies (consisting of VHH, CH2 and CH3 domains), humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgG1, IgG2, IgG3, IgG4, IgM, IgE, IgA, and IgD. Full length antibodies or whole antibody molecules are intact immunoglobulins comprising two immunoglobulin (Ig) heavy chains and two Ig light chains joined by disulfide bonds. An antibody according to the invention comprises an heavy chain comprising a heavy chain variable region.

By the term "specifically binds to" or "is specific for" as used herein with respect to an antibody, is meant an antibody or antibody fragment, which recognizes and binds to a specific antigen, but does not recognize or bind in significant amount (detectably bind) to other molecules present in the sample or to other molecules to which the antibody may come into contact in an organism, while detectably binding to its specific antigen. The specific binding of an antibody to its antigen may be determined by standard assays such as immunoassay. An antibody specifically binds to its antigen when it has a dissociation constant (KD) of 1 µM or less for its antigen in a standard KD determination assay. KD values are expressed as molar concentration (M); KD for antibodies are usually determined by Surface plasmon resonance using Biacore assay.

The term "Cytotoxic T-Lymphocyte Associated Protein 4" (CTLA4, CTLA-4, CD152) refers to a protein encoded by the *CTLA4* gene in a mammalian genome. CTLA4 is a member of the immunoglobulin superfamily and encodes a protein which transmits an inhibitory signal to T cells. The protein contains a V domain, a transmembrane domain, and a cytoplasmic tail. Alternate transcriptional splice variants, encoding different isoforms, have been characterized. The membrane-bound isoform functions as a homodimer interconnected by a disulfide bond, while the soluble isoform functions as a monomer. Representative examples of CTLA4 include without limitation, human (NCBI Gene ID: 1493), mouse (NCBI Gene ID: 12477) and other functional orthologs thereof. Human FAP has the 223 amino acid sequence UniProtKB/Swiss-Prot P16410.

CTLA-4 is a membrane receptor known to shuttle from the surface to the cytoplasm, where it can undergo endosomal degradation, or can be re-expressed by receptor recycling or by de-novo expression. This cycle defines an important mechanism of suppression of Treg cells that capture the CTLA-4 ligand CD80 from the surface of antigen-presenting cells and eliminate CD80 by trans-endocytosis and endosomal-mediated degradation (Qureshi et al, Science, 2011, 332, 600-603).

CTLA-4 is a receptor that is preferentially expressed on tumor-associated Treg. A receptor that is preferentially expressed on tumor-associated Treg refers to a cell-surface receptor that is overexpressed (or upregulated) on tumor-associated Treg as compared with peripheral Treg from blood, Tconv and CD8 T cells from tumor or blood, as illustrated in Figure 1.

The term interleukin-2 or IL-2, also known as TCGF or lymphokine, refers to a protein encoded by the IL-2 gene in a mammalian genome. IL-2 is expressed as a precursor containing a N-terminal signal peptide (20 amino acids) which is cleaved to yield the mature protein (IL-2). Representative examples of IL-2 include without limitation, human (Gene ID: 3558), rat (Gene ID: 116562), cat (Gene ID: 751114), and mouse (Gene ID 16183) forms. As used herein, IL-2, refers to wild-type IL-2. Human IL-2 precursor has the 153 amino acid sequence UniProtKB/Swiss-Prot: P60568.1. Mature IL-2 has the 133 amino acid sequence from positions 21 to 153 of the precursor and corresponds to SEQ ID NO: 1.

The term IL-2 mutein refers to a mutant IL-2 or IL-2 variant comprising one or more mutations (insertions, deletions, substitutions) as compared to wild-type IL-2. IL-2 mutein refers to IL-2 variant with modified biological activity as compared to wild-type IL-2. IL-2 mutein is preferably human IL-2 mutein.

The term Treg-specific IL-2 receptor antagonist or IL-2 mutein with IL-2 receptor antagonist activity specific for Tregs refers to an IL-2 mutein which is a dominant negative IL-2 molecule that does not induce IL-2-mediated STAT-5 phosphorylation in cells bearing the IL-2R and competes with wild-type IL-2 for signaling through the trimeric alpha, beta, gamma IL-2R. The IL-2 receptor antagonist activity specific for Tregs ot the IL-2 mutein according to the invention is illustrated in the examples of the present application.

As used herein, the term "functional" with respect to an anti-CTLA-4 antibody including variants thereof as disclosed herein, refers to an anti-CTLA-4 antibody which specifically binds to CTLA-4. The activity of the antibody can be determined by standard immunoassay with CTLA-4 protein or antigen thereof.

As used herein, the term "functional" with respect to an IL-2 mutein including variants thereof as disclosed herein, refers to an IL-2 mutein having IL-2 receptor antagonist activity specific for Tregs. The antagonist activity of the IL-2 mutein for IL-2 receptor can be determined by standard assays such as STATS phosphorylation assay in competition with Proleukin using a peripheral blood sample as disclosed in the examples.

As used herein, the term "functional" with respect to an immunocytokine according to the invention, refers to an immunocytokine which combines the CTLA-4 binding activity of the anti-CTLA-4 antibody and the IL-2 receptor antagonist activity specific for Tregs of the IL-2 mutein as disclosed herein and which further induces CD25 endocytosis as shown in the examples.

In the following description, the amino acid residues are designated by the standard one letter amino acid code. The indicated positions are determined by alignment with a reference sequence. The reference sequence for IL-2 mutein is human IL-2 sequence (SEQ ID NO: 1). For example, K9 is the lysine residue at position 9 of SEQ ID NO: 1. Substitutions are designated herein by the one letter amino acid code followed by the substituting residue in one letter amino acid code; K9E is a substitution of the lysine (K) residue at position 9 of SEQ ID NO: 1 with a Glutamic acid (E) residue.

As used herein, the term "variant" refers to a polypeptide comprising an amino acid sequence having at least 85% sequence identity with the native sequence. The term "variant" refers to a functional variant having the activity of the native sequence. The activity of a variant or fragment may be assessed using methods well-known by the skilled person such as disclosed in the examples of the present application.

The variant comprises one or more amino acid mutation(s), wherein the mutation may be insertion, deletion, or substitution with a conserved or non-conserved amino acid. Conservative substitutions are substitutions of one amino acid with another having similar chemical or physical properties (size, charge or polarity), which substitution generally does not adversely affect the biochemical, biophysical and/or biological properties of the protein (antibody or IL-2 mutein). Said conservative substitution(s) are advantageously chosen within one of the following five groups: Group 1-small aliphatic, non-polar or slightly polar residues (A, S, T, P, G); Group 2-polar, negatively charged residues and their amides (D, N, E, Q); Group 3-polar, positively charged residues (H, R, K); Group 4-large aliphatic, nonpolar residues (M, L, I, V, C); and Group 5-large, aromatic residues (F, Y, W).

The percent amino acid sequence or nucleotide sequence identity is defined as the percent of amino acid residues or nucleotides in a Compared Sequence that are identical to the Reference Sequence after aligning the sequences and introducing gaps if necessary, to achieve the maximum sequence identity and not considering any conservative substitutions for amino acid sequences as part of the sequence identity. Alignment for purposes of determining percent amino acid sequence identity can be achieved in various ways known to a person of skill in the art, for instance using publicly available computer software such as the GCG (Genetics Computer Group, Program Manual for the GCG Package, Version 7, Madison, Wisconsin) pileup program, or any of sequence comparison algorithms such as BLAST (Altschul et al., J. Mol. Biol., 1990, 215, 403-), FASTA or CLUSTALW. When using such software, the default parameters, e.g., for gap penalty and extension penalty, are preferably used. The BLASTP program uses as default a word length (W) of 3 and an expectation (E) of 10. Alignments are usually performed on the entire length of the reference sequence.

As used herein, the term "cancer" refers to any member of a class of diseases or disorders characterized by uncontrolled division of cells and the ability of these cells to invade other tissues, either by direct growth into adjacent tissue through invasion or by implantation into distant sites by metastasis. Metastasis is defined as the stage in which cancer cells are transported through the bloodstream or lymphatic system. The term cancer according to the present invention also comprises cancer metastases and relapse of cancer. Cancers are classified by the type of cell that the tumor resembles and, therefore, the tissue presumed to be the origin of the tumor. For example, carcinomas are malignant tumors derived from epithelial cells. This group represents the most common cancers, including the common forms of breast, prostate, lung, and colon cancer. Lymphomas and leukemias include malignant tumors derived from blood and bone marrow cells. Sarcomas are malignant tumors derived from connective tissue or mesenchymal cells. Mesotheliomas are tumors derived from the mesothelial cells lining the peritoneum and the pleura. Gliomas are tumors derived from glia, the most common type of brain cell. Germinomas are tumors derived from germ cells, normally found in the testicle and ovary. Choriocarcinomas are malignant tumors derived from the placenta. As used herein, "cancer" refers to any cancer type including solid and liquid tumors.

The terms "subject" and "patient" are used interchangeably herein and refer to both human and non-human animals. As used herein, the term "patient" denotes a mammal, such as with no limitations a rodent, a feline, a canine, a bovine, an ovine, an equine and a primate. Preferably, a patient according to the invention is a human.

The term "treating" or "treatment", as used herein, means reversing, alleviating, inhibiting the progress of, or preventing the disorder or condition to which such term applies, or reversing, alleviating, inhibiting the progress of, or preventing one or more symptoms of the disorder or condition to which such term applies. As used herein, the terms "treatment" or "treat" refer to both prophylactic or preventive treatment as well as curative or disease modifying treatment, including treatment of patients at risk of contracting the disease or suspected to have contracted the disease as well as patients who are ill or have been diagnosed as suffering from a disease or medical condition, and include suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment.

"Treating cancer" includes, without limitation, reducing the number of cancer cells or the size of a tumor in the patient, reducing progression of a cancer to a more aggressive form (i.e. maintaining the cancer in a form that is susceptible to a therapeutic agent), reducing proliferation of cancer cells or reducing the speed of tumor growth, killing of cancer cells, reducing metastasis of cancer cells or reducing the likelihood of recurrence of a cancer in a subject. Treating a subject as used herein refers to any type of treatment that imparts a benefit to a subject afflicted with cancer or at risk of developing cancer or facing a cancer recurrence. Treatment includes improvement in the condition of the subject (e.g., in one or more symptoms), delay in the progression of the disease, delay in the onset of symptoms, slowing the progression of symptoms and others.

"a", "an", and "the" include plural referents, unless the context clearly indicates otherwise. As such, the term "a" (or "an"), "one or more" or "at least one" can be used interchangeably herein; unless specified otherwise, "or" means "and/or".

### Immunocytokine

The invention relates to a fusion protein comprising an anti-CTLA-4 antibody and an IL-2 variant which is a regulatory T cell specific IL-2 receptor antagonist comprising the substitutions K9E, L12E, H16R, L19R, M23L, N26K, S87N, V91K, E95K, N119K, T123A; and further comprising the subsitutions S127K; Y31P and S127K; or K49Q, E52S, R81E, D84N, T131R and L132S, the indicated positions being determined by alignment with human IL-2 (SEQ ID NO: 1), and wherein the N-terminus of the IL-2 variant is fused to the C-terminus of the antibody heavy chain.

The protein according to the invention which is a fusion of an anti-CTLA-4 antibody to a variant of IL-2 cytokine is an immunocytokine. The regulatory T cell specific IL-2 receptor antagonist is named herein IL-2 antagonist or IL-2 mutein.

The immunocytokine according to the invention induces CD25 endocytosis and Treg apoptosis as shown in the examples of the present application. These properties allow to deplete selectively tumor-associated Tregs.

The immunocytokine according to the invention may use known anti-CTLA-4 antibodies, in particular therapeutic anti-CTLA-4 antibodies. Alternatively, new anti-CTLA-4 antibodies may be obtained by standard methods that are well-known in the art as disclosed herein.

For example, antibodies of the present disclosure can be produced by immunization of a laboratory animal with an antigen (receptor protein or fragment thereof, eventually coupled to a carrier) to induce the production of antibodies by the B cells of said mammal; and recovery of the antibodies from the serum of the immunized animal. To obtain monoclonal antibodies, B cells are isolated from the spleen of the immunized animals and immortalized according to standard hybridoma production techniques. Antibodies of the present disclosure can also be obtained by screening of a phage display library. In particular, VH and VL fragments of the antibodies may be screened from a phage display library using a peptide antigen and recombinant antibodies may be produced according to standard techniques which are well-known in the art. Antibody VH and VL sequences may be obtained by single-cell B-cell receptor sequencing using high throughput sequencing techniques as disclosed in Goldstein et al., Communications Biology, 2019, 2, 304. Antibodies of the present disclosure can also be produced in a host cell transfectoma using, for example, a combination of recombinant DNA techniques and gene transfection methods as is well known in the art (Morrison, Science, 1985, 229, 1202-1207). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. When recombinant expression vectors encoding antibody genes are introduced into host cells, in particular eukaryotic cells such as mammalian cells, the antibodies are produced by culturing the host cells for a period of time sufficient for expression of the antibody in the host cells and, optionally, secretion of the antibody into the culture medium in which the host cells are grown. Antibodies can be recovered and purified for example from the culture medium after their secretion using standard protein purification methods (Shukla et al., Journal of Chromatography, 2007, 848, 28-39).

The antibody as defined in the invention binds specifically to the CTLA-4 receptor that is preferentially expressed on tumor-associated Treg. In some embodiments, the antibody may further inhibit signaling through the receptor, which means that the antibody is a bloking, inhibitory or antagonist antibody.

In some embodiments, the antibody comprises a heavy chain variable region (VH) and a light chain variable region (VL), preferably human or humanized VH and VL. In some particular embodiments, the antibody further comprises an Ig constant region, including native sequence Ig constant region and variant Ig constant region. The Ig constant region is preferably human. The Ig constant region comprises at least one Ig constant domain; in particular at least CH1 and CL, more particularly CL and CH1 and further Fc domain (CH2 and/or CH3 domains). The Fc region may be from any Ig class or isotype, preferably human IgG isotype. The Fc region may be native sequence Fc region or variant Fc region. An example of variant Fc region is a silent Fc region; silent IgG1 Fc may comprise N297A or L234A and L235A mutations. Another example of variant Fc region comprises mutations to enhance ADCC, in particular mutations to enhance to FcyR affinity such as S298A/E333A/K334A; S239D/I332E; P247I/A339Q (Review in van der Horst et al., Cancers, 2020, 12, 3041). Variant human Fc constant region may comprise the M428L and N434S substitutions (LS) to enhance antibody half-life The numbering of residues in the Fc region is that of the EU index of Kabat.

In some particular embodiments, the antibody comprises a human IgG1 Fc domain and/or a human Ig kappa light chain constant domain.

In some particular embodiments, the antibody is a full-length human or humanized antibody. The antibody is preferably an IgG, particularly IgG1. The corresponding immunocytokine has the classical Ig structure comprising two Ig heavy chains and two Ig light chains and a copy of IL-2 variant fused to each heavy chain C-terminal end.

In some embodiments, the antibody is a single-domain antibody or nanobody such as VHH.

In some embodiments, the antibody is a heavy chain-only antibody in particular fully human heavy chain-only antibody.

Various anti-CTLA-4 antibodies have been described in the art and can be used in the immunocytokine according to the invention. Non-limiting examples of known anti-CTLA4 antibodies including monoclonal antibodies, as well as chimeric, fully human and humanized versions thereof; and heavy chain-only antibody in particular fully human heavy chain-only antibody are disclosed in the following references: ipilimumab (Patents US 7,605,238; US6,984,720 and US8,017,114); tremelimumab (Patent US 6,682 736; US 7,109,003 and US 8,143,379); International patent applications WO 0037504; WO 01/14424; WO 2019/174603 ; WO 2021/129775 ; WO 2021/13102; WO 2022/017428; WO 2022/169269; WO 2022/184155; WO 2022/184155; WO 2022/222961; patent US8,491,895; single chain anti-CTLA4 antibodies (International patent applications WO 1997/020574, WO 2007/123737, WO 2019/233413; Gan et al., PNAS, 2022, 119, e2200879119); Oncology Live, June 22, 2023, 24, 11, 39-; J Clin Oncol. 2023;41(suppl 16)). Other anti-CTLA4 antibodies that may be used in the immunocytokine of the invention include: ADG116 ; Botensilimab (AGEN1181); BMS-986249 and BMS-986288; HBM4003; Quavonlimab (MK-1308; Perets et al., Ann Oncol. 2021;32(3):395-403. doi:10.1016/j.annonc.2020.11.020); Zalifrelimab (AGEN1884); Gotistobart (ONC-392) ; XmAb22841 and XTX101; and the anti-CTLA4 antibodies shown in Table 3. Alternatively, new anti-CTLA4 antibodies may be obtained by standard methods that are well-known in the art as disclosed herein.

In some particular embodiments, the anti-CTLA-4 antibody is a therapeutic antibody such as Ipilimumab (human IgG1), tremelimumab (human IgG2a) or a functional variant thereof.

In some particular embodiments, the anti-CTLA-4 antibody comprises a H-CDR1 of SEQ ID NO: 2, a H-CDR2 of SEQ ID NO: 3, a H-CDR3 of SEQ ID NO: 4, a L-CDR1 of SEQ ID NO: 5, a L-CDR2 of SEQ ID NO: 6 and a L-CDR3 of SEQ ID NO: 7 or variants further having one or more conservative substitutions on one or more of these CDRs. In particular, it is contemplated that monoclonal antibodies or antigen-binding fragment thereof may have 1, 2, 3, 4, 5, 6, or more alterations in the amino acid sequence of 1, 2, 3, 4, 5, or 6 CDRs of monoclonal antibodies provided herein, in particular in the CDR of SEQ ID NO: 2 to 7. In some particular embodiments, the anti-CTLA-4 antibody comprises a VH domain having at least 85 % identity with SEQ ID NO: 8 and a VL domain having at least 85 % identity with SEQ ID NO: 9. Preferably, said VH and/or VH domain has at least 86%, 87%, 88%, 89%, 90%, 91%, 92%, 93%, 94%, 95%, 96%, 97%, 98% or 99% identity with said sequence, even more preferably at least 95%, 96%, 97%, 98% or 99% 98% or 99% identity with said sequence. Preferably, said VH domain has a H-CDR1 of SEQ ID NO: 2, a H-CDR2 of SEQ ID NO: 3, and a H-CDR3 of SEQ ID NO: 4 and/or said VL domain has a L-CDR1 of SEQ ID NO: 5, a L-CDR2 of SEQ ID NO: 6 and a L-CDR3 of SEQ ID NO: 7.

In some particular embodiments, the anti-CTLA-4 antibody comprises an IgG Fc region, including native sequence Fc region and variant Fc regions as disclosed herein. In some particular embodiments, the anti-CTLA-4 antibody comprises a human IgG1 Fc domain and/or a human Ig kappa light chain constant domain.

In some particular embodiments, the anti-CTLA-4 antibody is a single-domain antibody or nanobody such as VHH.

In some particular embodiments, the anti-CTLA-4 antibody is a heavy chain-only antibody in particular fully human heavy chain-only antibody. In some particular embodiments, the anti-CTLA-4 antibody is bispecific or polyspecific. For example, the antibody may comprise an antigen-binding site specific for another protein, in particular another surface molecule specific for Tregs as disclosed herein. More particularly a surface molecule preferentially expressed on tumor-associated Tregs. The antibody specific for another protein may target any receptor that is preferentially expressed on tumor-associated Tregs. Examples of such receptor are known in the art and disclosed for example in WO 2021/165546. Any of the cell-surface receptor of tumor-specific Tregs disclosed in WO 2021/165546 may be targeted by a bispecific or polyspecic antibody according to the present invention, in particular the cell-surface receptors disclosed in Table 1 or Table 2 of WO 2021/165546.

In the various embodiments, the IL-2 mutein may comprise the substitutions : K9E, L12E, H16R, L19R, M23L, N26K, S87N, V91K, E95K, N119K, T123A and S127K; K9E, L12E, H16R, L19R, M23L, N26K, Y31P, S87N, V91K, E95K, N119K, T123A and S127K; or K9E, L12E, H16R, L19R, M23L, N26K, K49Q, E52S, R81E, D84N, S87N, V91K, E95K, N119K, T123A, T131R and L132S. In some preferred embodiments, the IL-2 mutein comprises the substitutions K9E, L12E, H16R, L19R, M23L, N26K, Y31P, S87N, V91K, E95K, N119K, T123A and S127K.

In the various embodiments, the IL-2 mutein may comprise at least one additional amino acid mutation (insertion, deletion, substitution) or not. In some embodiments, the IL-2 mutein does not comprise additional amino acid mutations.

In some other embodiments, the IL-2 mutein comprises at least one additional amino acid mutation (insertion, deletion, substitution). The IL-2 mutein comprises preferably at least one amino acid deletion, more preferably at a position selected from S4, S5 or S6, the indicated positions being determined by alignment with SEQ ID NO: 1. The amino acid deletion may be combined or replaced with at least one additional substitution chosen from : a 3T to A amino acid substitution, to control for potential O-glycosylation in the T residue; and a 125 C to A substitution, to control for free 125 cysteine that may induce di-sulfide bounds. The IL-2 mutein can comprise 1, 2 or all of the deletions and/or additional substitutions listed above in combination with the above listed substitutions. All of these possible combinations are specifically contemplated. The IL-2 mutein is at least 125 amino acids in size. Preferably, the IL-2 variant is 125 to 135 amino acids in size. In some preferred embodiments, the IL-2 mutein is human IL-2 mutein.

In some particular embodiments, the IL-2 mutein has at least 85% amino acid identity with SEQ ID NO: 1. Preferably, said IL-2 mutein has at least 86%, 87%, 88%, 89%, 90%, 91%, 92%, 93%, 94%, 95%, 96%, 97%, 98% or 99% identity with said sequence, even more preferably at least 95%, 96%, 97%, 98% or 99% 98% or 99% identity with said sequence.

In some preferred embodiments, the IL-2 mutein does not comprise any substitution at positions: 11, 13, 15, 18, 20, 22, 29, 30, 35, 37, 48, 68, 69, 71, 74, 75, 76, 80, 85, 86, 88, 92, 110, 126, 129, 130 and 133; 11, 13, 15, 18, 20, 22, 29, 30, 35, 37, 48, 68, 69, 71, 74, 75, 76, 80, 85, 86, 88, 92, 110, 125, 126, 129, 130 and 133 or at positions 4, 8, 10, 11, 13, 15, 18, 20, 22, 29, 30, 35, 37, 38, 42, 45, 48, 62, 67, 68, 69, 71, 74, 75, 76, 80, 85, 86, 88, 90, 92, 110, 125, 126, 128, 129, 130 and 133.

In some preferred embodiments, the IL-2 mutein has at least 85%, 86%, 87%, 88%, 89%, 90%, 91%, 92%, 93%, 94%, 95%, 96%, 97%, 98% or 99% identity with any one of SEQ ID NO: 13, 14, or 15; preferably at least 95%, 96%, 97%, 98% or 99% 98% or 99% identity with any one of SEQ ID NO: 13, 14, or 15; more preferably at least 85%, 86%, 87%, 88%, 89%, 90%, 91%, 92%, 93%, 94%, 95%, 96%, 97%, 98% or 99% identity with SEQ ID NO: 14; even more preferably at least 95%, 96%, 97%, 98% or 99% 98% or 99% identity with SEQ ID NO: 14; more preferably, said IL-2 mutein does not comprise any substitution at positions: 11, 13, 15, 18, 20, 22, 29, 30, 35, 37, 48, 68, 69, 71, 74, 75, 76, 80, 85, 86, 88, 92, 110, 126, 129, 130 and 133; 11, 13, 15, 18, 20, 22, 29, 30, 35, 37, 48, 68, 69, 71, 74, 75, 76, 80, 85, 86, 88, 92, 110, 125, 126, 129, 130 and 133 or at positions 4, 8, 10, 11, 13, 15, 18, 20, 22, 29, 30, 35, 37, 38, 42, 45, 48, 62, 67, 68, 69, 71, 74, 75, 76, 80, 85, 86, 88, 90, 92, 110, 125, 126, 128, 129, 130 and 133.

The IL-2 mutein is fused to the C-terminus of the antibody heavy chain, which means that the N-terminus of the IL-2 mutein is fused to the C-terminus of the antibody heavy chain. The IL-2 mutein may be fused to the antibody directly or via a linker or spacer that is used to physically separate two protein domains. Suitable linkers are known in the art and include for example linkers comprising Glycine and Serine residues and others. An example of suitable linker comprises the sequence (GGGGS)n, wherein n is preferably no more than 10 (1, 2, 3, 4, 5, 6,7, 8, 9, 10). In some embodiments, the fusion protein comprises a linker of sequence (GGGGS)3, corresponding to SEQ ID NO: 10.

In some particular embodiments, the fusion protein according to the invention is derived from a whole antibody molecule, preferably whole human or humanized antibody molecule, such as human or humanized IgG1 and IgG4 and the N-terminus of the IL-2 mutein is fused to the C-terminus of the antibody heavy chain via a linker such as (GGGGS)n, more preferably (GGGGS)3.

In some preferred embodiments, the fusion protein according to the invention comprises:
- an anti-CTLA-4 antibody heavy chain fused to the IL-2 variant, wherein said heavy chain fusion has at least 85%, 90%, 95% or 98% sequence identity with SEQ ID NO: 11; and
- an anti-CTLA-4 antibody light chain having at least 85%, 90%, 95% or 98% sequence identity with SEQ ID NO: 12.

Preferably, the VH domain of the anti-CTLA-4 antibody heavy chain has a H-CDR1 of SEQ ID NO: 2, a H-CDR2 of SEQ ID NO: 3, and a H-CDR3 of SEQ ID NO: 4 and/or the VL domain of the anti-CTLA-4 antibody light chain has a L-CDR1 of SEQ ID NO: 5, a L-CDR2 of SEQ ID NO: 6 and a L-CDR3 of SEQ ID NO: 7.

The fusion protein may comprise an additional moiety and/or may be further modified. The invention encompasses immunocytokines having one or more chemical modifications into one or more amino acid residues, peptide bonds, N-and/or C-terminal ends, as long as the modified immmunocytokine is functional. These modifications which are introduced into the fusion protein by the conventional methods known to those skilled in the art, include, in a non-limiting manner: coupling to a molecule or agent of interest, for example PEG (pegylation) to increase the biological (e.g., serum) half-life of the immunocytokine; substitution of a natural amino acid with a non-proteinogenic amino acid (D amino acid or amino acid analog); modification of the peptide bond, in particular with a bond of the retro or retro-inverso type or a bond different from the peptide bond; the cyclization, and the addition of a chemical group to the amino acid side chain or the N-and/or C-terminal end(s) of the immunocytokine, in particular for coupling to a molecule or agent of interest to the immunocytokine.

In some embodiments the IL-2 variant is provided in a form where it is associated with at least an agent of interest, for example in the form of a complex such as a molecular complex or a particle or a conjugate. The agent of interest includes with no limitation any therapeutic agent including a cell such as patient's Chimeric Antigen Receptor (CAR) T-cell.

The term immunocytokine as used herein encompasses the different forms of immunocytokines disclosed herein, such as an immunocytokine, modified or not, associated or not with at least an agent of interest in the form of a complex or conjugate as disclosed above.

The immunocytokine according to the invention can be made by routine techniques in the art, in particular by expression of a recombinant DNA in a suitable cell system (eukaryotic or prokaryotic) and screened for activity (i.e. capable of inducing CD25 endocytosis and/or inducing Treg apoptosis ) using the assays described herein or other similar assays.

### Nucleic acid, vector, host cell and immunocytokine production

Also disclosed herein are nucleic acid molecules that encode the immunocytokine of the present disclosure.

Typically, said nucleic acid is recombinant, synthetic, or semi-synthetic nucleic acid which is expressible in a host cell suitable for immunocytokine expression or production, in particular human immunocytokine production. The host cell may be a cell for recombinant immunocytokine production or a patient cell for immunocytokine production *in vivo.* Typically, the nucleic acid may be DNA, RNA, or mixed molecule, which may further be modified and/or included in any suitable expression vector. As used herein, the terms "vector" and "expression vector" mean the vehicle by which a DNA or RNA sequence (e.g. a foreign gene) can be introduced into a host cell, so as to transform the host and promote expression (e.g. transcription and translation) of the introduced sequence. The recombinant vector can be a vector for eukaryotic or prokaryotic expression, such as a plasmid, a phage for bacterium introduction, a YAC able to transform yeast, a viral vector and especially a retroviral vector, or any expression vector. An expression vector as defined herein is chosen to enable the production of an immunocytokine, either *in vitro* or *in vivo.*

Examples of nucleic acid molecules are those encoding the amino acid sequence(s) of the immunocytokine as disclosed in the previous section. In some particular embodiments, said nucleic acid molecules encode the heavy and light chain amino acid sequences of the immunocytokine as disclosed in the previous section. In some more particular embodiments, said nucleic acid molecules are selected from the pair of nucleotide sequences SEQ ID NO: 16 and 17 which encodes the immunocytokine comprising the heavy chain of SEQ ID NO: 11 and the light chain of SEQ ID NO: 12.

Nucleic acids encoding immunocytokine of the disclosure with nucleotide sequences having at least 80%, for example, at least 85%, 90%, 95%, 96%, 97%, 98%, or 99% identity to any one of the above defined nucleotides sequences are also part of the present disclosure.

The present disclosure also pertains to nucleic acid molecules or constructs that derive from the latter sequences having been optimized for protein expression in host cells, in particular eukaryotic cells, preferably mammalian cells, for example, CHO or HEK cell lines or human cells. Codon optimization is used to improve protein expression level in living organism by increasing translational efficiency of target gene. Appropriate methods and softwares for codon optimization in the desired host are well-known in the art and publicly available (see for example the GeneOptimizer software suite in Raab et al., Systems and Synthetic Biology, 2010, 4, (3), 215-225).

In some embodiments, said nucleic acid molecule is a eukaryotic, preferably mammalian, expression cassette, wherein the immunocytokine coding sequence(s) is operably linked to appropriate regulatory sequence(s) for their expression in an immunocytokine producing cell or a patient cell. Such sequences which are well-known in the art include in particular a promoter, and further regulatory sequences capable of further controlling the expression of a transgene, such as without limitation, enhancer, terminator and intron. The promoter may be a tissue-specific, ubiquitous, constitutive, or inducible promoter that is functional in the immunocytokine producing cell. Such promoters are well-known in the art and their sequences are available in public sequence data bases.

A further object of the disclosure relates to a vector comprising at least one nucleic acid encoding the immunocytokine of the present disclosure.

The invention may use any vector suitable for the delivery and expression of nucleic acid into individual's cells, in particular suitable for nucleic acid therapy. Such vectors that are well-known in the art include viral and non-viral vectors. Non-viral vector includes the various (non-viral) agents which are commonly used to either introduce or maintain nucleic acid into individual's cells. Agents which are used to introduce nucleic acid into individual's cells by various means include in particular polymer-based, particle-based, lipid-based, peptide-based delivery vehicles or combinations thereof, such as with no limitations cationic polymer, dendrimer, micelle, liposome, lipopolyplex, exosome, microparticle and nanoparticle including lipid nanoparticle (LNP) and viral-like particles; and cell penetrating peptides (CPP). Agents which are used to maintain nucleic acid into individual's cells include in particular naked nucleic acid vectors such as plasmids, transposons and mini-circles. Viral vectors are by nature capable of penetrating into cells and delivering nucleic acid(s) of interest into cells, according to a process named as viral transduction. As used herein, the term "viral vector" refers to a non-replicating, non-pathogenic virus engineered for the delivery of genetic material into cells. In viral vectors, viral genes essential for replication and virulence are replaced with an expression cassette for the transgene of interest. Thus, the viral vector genome comprises the transgene expression cassette flanked by the viral sequences required for viral vector production. As used herein, the term "recombinant virus" refers to a virus, in particular a viral vector, produced by standard recombinant DNA technology techniques that are known in the art. As used herein, the term "virus particle" or "viral particle" is intended to mean the extracellular form of a non-pathogenic virus, in particular a viral vector, composed of genetic material made from either DNA or RNA surrounded by a protein coat, called the capsid, and in some cases an envelope derived from portions of host cell membranes and including viral glycoproteins. As used herein, a viral vector refers to a viral vector particle. These vectors have minimal eukaryotic sequences to minimize the possibility of chromosomal integration. In addition, these approaches can advantageously be combined to introduce and maintain the nucleic acid of the invention into individual's cells.

In particular embodiments, the vector is a particle or vesicle, in particular lipid-based micro- or nano-vesicle or particle such as liposome or lipid nanoparticle (LNP). In more particular embodiments, the nucleic acid is RNA, in particular mRNA and the vector is a particle or vesicle, in particular LNP as described above.

In other embodiments, the nucleic acid is DNA, preferably included in an expression vector such as plasmid or viral vector. The vector may be a recombinant integrating or non-integrating viral vector. Examples of recombinant viral vectors include, but not limited to, vectors derived from retrovirus, adenovirus, adeno-associated virus (AAV), herpes virus, poxvirus, and other viruses. Retroviruses includes in particular lentivirus vector such as human immunodeficiency virus, including HIV type 1 (HIV1) and HIV type 2 (HIV2) vectors.

The polynucleotide according to the disclosure is prepared by the conventional methods known in the art. For example, it is produced by amplification of a nucleic sequence by PCR or RT-PCR, by screening genomic DNA libraries by hybridization with a homologous probe, or else by total or partial chemical synthesis. The recombinant vectors are constructed and introduced into host cells by the conventional recombinant DNA and genetic engineering techniques, which are known in the art.

A further object of the present disclosure relates to a host cell which has been transfected, infected or transformed by a nucleic acid and/or a vector according to the invention. As used herein, the term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. The transformation may be transient or stable over time. Stable transformation may be by integration of the nucleic acid into the host cell genome. A host cell that receives and expresses introduced DNA or RNA bas been "transformed".

Said host cells may be prokaryotic cells such as bacteria or eukaryotic cells such as yeasts, insect cells or mammalian cells. Mammalian cells may be simian, human, dog, and rodent cells. Mammalian host cells for expressing the antibodies of the disclosure include in particular Chinese Hamster Ovary (CHO cells) including dhfr- CHO cells (described in Urlaub and Chasin, 1980) used with a DHFR selectable marker (as described in Kaufman and Sharp, 1982), CHOK1 dhfr+ cell lines. In a preferred embodiment, said host cells are CHO cells. In another preferred embodiment, said host cells are insect cells.

The polynucleotide, vector or cell of the disclosure are useful for the production of the protein of the invention using well-known recombinant DNA techniques. The polynucleotide or vector are also useful for nucleic acid therapy as disclosed below.

Another aspect of the invention relates to a method of production of an immunocytokine according to the present disclosure, comprising: (i) culturing the host cell according to the present disclosure for expression of said immunocytokine by the host cell; and optionally (ii) recovering the immunocytokine; and (iii) purifying said immunocytokine.

Immunocytokines of the present disclosure can be produced in a host cell transfectoma using, for example, a combination of recombinant DNA techniques and gene transfection methods as is well known in the art (Morrison et al., Science, 1985, 229, 1202-1207). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. When recombinant expression vectors encoding immunocytokine genes are introduced into host cells, in particular eukaryotic cells such as mammalian cells, the immunocytokine is produced by culturing the host cells for a period of time sufficient for expression of the immunocytokine in the host cells and, optionally, secretion of the immunocytokine into the culture medium in which the host cells are grown. Immunocytokines can be recovered and purified for example from the culture medium after their secretion using standard protein purification methods (Shukla et al., J. Chromatogr. B. Analyt. Technol. Biomed. Life Sci., 2007, 848, 28-39). Methods for purifying polypeptides are well known in the art, such as chromatography (e.g., ion exchange chromatography, gel permeation chromatography and reversed phase chromatography).

### Pharmaceutical composition and therapeutic use

The immunocytokine, polynucleotide, and/or vector according to the invention are used for treating cancer.

The immunocytokine, polynucleotide and/or vector according to the invention are administered to the patient and the tumor infiltrating Tregs, in particular tumor-associated Tregs are inhibited or eliminated *in vivo* in the patient, unleashing immune cells (B, NK, CD4+ or CD8+ T cells; DC; macrophages and others) from Treg suppression. The immunocytokine, polynucleotide, vector and/or cell according to the invention are used to selectively deplete tumor infiltrating Tregs, in particular tumor-associated Tregs by depriving them from IL-2 while leaving effector immune cells, in particular NK cells, CD4+ and CD8+ T cells untouched and allowing their expansion. In particular embodiments, the immunocytokine, polynucleotide, vector and/or cell according to the invention are used to selectively deplete tumor infiltrating Tregs, in particular tumor-associated Tregs. In particular embodiments, the immunocytokine, polynucleotide, vector and/or cell according to the invention are used to stimulate an anti-tumoral CD8+ T-cell response.

The present invention relates to a pharmaceutical composition comprising, as active substance, an immunocytokine, polynucleotide, and/or vector according to the invention, and at least one pharmaceutically acceptable vehicle and/or carrier.

A "pharmaceutically acceptable carrier" refers to a vehicle in which the therapeutic is administered and that does not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

Preferably, the pharmaceutical composition contains vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or suspensions. The solution or suspension may comprise additives which are compatible with the active agent. In all cases, the form must be sterile and must be fluid to the extent that easy syringe ability exists. It must be stable under the conditions of manufacture and storage and must be preserved against the contaminating action of microorganisms, such as bacteria and fungi. An example of an appropriate solution is a buffer, such as phosphate buffered saline (PBS) or Ringer lactate.

The pharmaceutical composition is formulated for administration by a number of routes, including but not limited to oral, parenteral and local. The pharmaceutical vehicles are those appropriate to the planned route of administration, which are well known in the art. Compositions can take the form of one or more dosage units.

In some particular embodiments, the pharmaceutical composition is a liquid preparation, preferably comprising a concentrated liquid formulation of the immunocytokine. In some other particular embodiments, the pharmaceutical composition is a lyophilized preparation.

The pharmaceutical composition comprises a therapeutically effective amount of the immunocytokine, polynucleotide and/or vector. In the context of the invention a therapeutically effective amount refers to a dose sufficient for reversing, alleviating or inhibiting the progress of the disorder or condition to which such term applies, or reversing, alleviating or inhibiting the progress of one or more symptoms of the disorder or condition to which such term applies. The term "effective dose" or "effective dosage" is defined as an amount sufficient to achieve, or at least partially achieve, the desired effect. A therapeutically effective amount is sufficient to show a positive medical response in the individual to whom it is administered. A positive medical response refers to the reduction of subsequent (preventive treatment) or established (therapeutic treatment) disease symptoms. The positive medical response comprises a partial or total inhibition of the symptoms of the disease. A positive medical response can be determined by measuring various objective parameters or criteria such as objective clinical signs of the disease and/or the increase of survival. A medical response to the composition according to the invention can be readily verified in appropriate animal models of the disease which are well-known in the art.

The effective dose is determined and adjusted depending on factors such as the composition used, the route of administration, the physical characteristics of the individual under consideration such as sex, age and weight, concurrent medication, and other factors, that those skilled in the medical arts will recognize. The effective dose can be determined by standard clinical techniques. In addition, *in vivo* and/or *in vitro* assays may optionally be employed to help predict optimal dosage ranges.

In some particular embodiments, the therapeutically effective amount of the immunocytokine is at least 0.05 mg/kg .

In some embodiments, the pharmaceutical composition comprises another active agent wherein said active agent is a pharmaceutical agent or therapeutic capable of preventing, treating or ameliorating cancer in humans or animals. The active agent may be a protein including an antibody, an oligonucleotide including an antisense oligonucleotide, peptide nucleic acid (PNA), small interfering RNA, locked nucleic acids (LNA), phosphorodiamidate morpholino oligonucleotides (PMO) and decoy DNA molecule, a plasmid, an aptamer including DNA, RNA or peptide aptamer, a small or large chemical drug, or mixtures thereof. In particular, the active agent may be an immunomodulatory agent such as immune checkpoint inhibitor. The active agent may also be an anticancer agent or a tumor antigen.

The invention provides also an immunocytokine, polynucleotide, vector or pharmaceutical composition according to the invention for use as a medicament or for use in therapy.

The invention provides also an immunocytokine, polynucleotide, vector, or pharmaceutical composition according to the invention for use in the prevention or treatment of cancer.

The invention provides also the use of an immunocytokine, polynucleotide, vector, or pharmaceutical composition according to the invention for the prevention or treatment of cancer.

The invention provides also the use of an immunocytokine, polynucleotide, vector, or pharmaceutical composition according to the invention in the manufacture of a medicament for the prevention or treatment of cancer.

The invention provides also a pharmaceutical composition for treatment of cancer, comprising an immunocytokine, polynucleotide, and/or vector according to the invention as an active component.

The invention provides also a pharmaceutical composition comprising an immunocytokine, polynucleotide, and/or vector according to the invention for treating cancer. As used herein, the term "cancer" refers to any cancer that may affect any one of the following tissues or organs: breast; liver; kidney; heart, mediastinum, pleura; floor of mouth; lip; salivary glands; tongue; gums; oral cavity; palate; tonsil; larynx; trachea; bronchus, lung; pharynx, hypopharynx, oropharynx, nasopharynx; esophagus; digestive organs such as stomach, intrahepatic bile ducts, biliary tract, pancreas, small intestine, colon; rectum; urinary organs such as bladder, gallbladder, ureter; rectosigmoid junction; anus, anal canal; skin; bone; joints, articular cartilage of limbs; eye and adnexa; brain; peripheral nerves, autonomic nervous system; spinal cord, cranial nerves, meninges; and various parts of the central nervous system; connective, subcutaneous and other soft tissues; retroperitoneum, peritoneum; adrenal gland; thyroid gland; endocrine glands and related structures; female genital organs such as ovary, uterus, cervix uteri; corpus uteri, vagina, vulva; male genital organs such as penis, testis and prostate gland; hematopoietic and reticuloendothelial systems; blood; lymph nodes; thymus.

The term "cancer" according to the invention comprises leukemias, seminomas, melanomas, teratomas, lymphomas, non-Hodgkin lymphoma, neuroblastomas, gliomas, adenocarcinoma, mesothelioma (including pleural mesothelioma, peritoneal mesothelioma, pericardial mesothelioma and end stage mesothelioma), rectal cancer, endometrial cancer, thyroid cancer (including papillary thyroid carcinoma, follicular thyroid carcinoma, medullary thyroid carcinoma, undifferentiated thyroid cancer, multiple endocrine neoplasia type 2A, multiple endocrine neoplasia type 2B, familial medullary thyroid cancer, pheochromocytoma and paraganglioma), skin cancer (including malignant melanoma, basal cell carcinoma, squamous cell carcinoma, Kaposi's sarcoma, keratoacanthoma, moles, dysplastic nevi, lipoma, angioma and dermatofibroma), nervous system cancer, brain cancer (including astrocytoma, medulloblastoma, glioma, lower grade glioma, ependymoma, germinoma (pinealoma), glioblastoma multiform, oligodendroglioma, schwannoma, retinoblastoma, congenital tumors, spinal cord neurofibroma, glioma or sarcoma), skull cancer (including osteoma, hemangioma, granuloma, xanthoma or osteitis deformans), meninges cancer (including meningioma, meningiosarcoma or gliomatosis), head and neck cancer (including head and neck squamous cell carcinoma and oral cancer (such as, e.g., buccal cavity cancer, lip cancer, tongue cancer, mouth cancer or pharynx cancer)), lymph node cancer, gastrointestinal cancer, liver cancer (including hepatoma, hepatocellular carcinoma, cholangiocarcinoma, hepatoblastoma, angiosarcoma, hepatocellular adenoma and hemangioma), colon cancer, stomach or gastric cancer, esophageal cancer (including squamous cell carcinoma, larynx, adenocarcinoma, leiomyosarcoma or lymphoma), colorectal cancer, intestinal cancer, small bowel or small intestines cancer (such as, e.g., adenocarcinoma lymphoma, carcinoid tumors, Kaposi's sarcoma, leiomyoma, hemangioma, lipoma, neurofibroma or fibroma), large bowel or large intestines cancer (such as, e.g., adenocarcinoma, tubular adenoma, villous adenoma, hamartoma or leiomyoma), pancreatic cancer (including ductal adenocarcinoma, insulinoma, glucagonoma, gastrinoma, carcinoid tumors or vipoma), ear, nose and throat (ENT) cancer, breast cancer (including HER2-enriched breast cancer, luminal A breast cancer, luminal B breast cancer and triple negative breast cancer), cancer of the uterus (including endometrial cancer such as endometrial carcinomas, endometrial stromal sarcomas and malignant mixed Müllerian tumors, uterine sarcomas, leiomyosarcomas and gestational trophoblastic disease), ovarian cancer (including dysgerminoma, granulosa-theca cell tumors and Sertoli-Leydig cell tumors), cervical cancer, vaginal cancer (including squamous-cell vaginal carcinoma, vaginal adenocarcinoma, clear cell vaginal adenocarcinoma, vaginal germ cell tumors, vaginal sarcoma botryoides and vaginal melanoma), vulvar cancer (including squamous cell vulvar carcinoma, verrucous vulvar carcinoma, vulvar melanoma, basal cell vulvar carcinoma, Bartholin gland carcinoma, vulvar adenocarcinoma and erythroplasia of Queyrat), genitourinary tract cancer, kidney cancer (including clear renal cell carcinoma, chromophobe renal cell carcinoma, papillary renal cell carcinoma, adenocarcinoma, Wilms tumor, nephroblastoma, lymphoma or leukemia), adrenal cancer, bladder cancer, urethra cancer (such as, e.g., squamous cell carcinoma, transitional cell carcinoma or adenocarcinoma), prostate cancer (such as, e.g., adenocarcinoma or sarcoma) and testis cancer (such as, e.g., seminoma, teratoma, embryonal carcinoma, teratocarcinoma, choriocarcinoma, sarcoma, interstitial cell carcinoma, fibroma, fibroadenoma, adenomatoid tumors or lipoma), lung cancer (including small cell lung carcinoma (SCLC), non-small cell lung carcinoma (NSCLC) including squamous cell lung carcinoma, lung adenocarcinoma (LUAD), and large cell lung carcinoma, bronchogenic carcinoma, alveolar carcinoma, bronchiolar carcinoma, bronchial adenoma, lung sarcoma, chondromatous hamartoma and pleural mesothelioma), sarcomas (including Askin's tumor, sarcoma botryoides, chondrosarcoma, Ewing's sarcoma, malignant hemangioendothelioma, malignant schwannoma, osteosarcoma and soft tissue sarcomas), soft tissue sarcomas (including alveolar soft part sarcoma, angiosarcoma, cystosarcoma phyllodes, dermatofibrosarcoma protuberans, desmoid tumor, desmoplastic small round cell tumor, epithelioid sarcoma, extraskeletal chondrosarcoma, extraskeletal osteosarcoma, fibrosarcoma, gastrointestinal stromal tumor (GIST), hemangiopericytoma, hemangiosarcoma, Kaposi's sarcoma, leiomyosarcoma, liposarcoma, lymphangiosarcoma, lymphosarcoma, malignant peripheral nerve sheath tumor (MPNST), neurofibrosarcoma, plexiform fibrohistiocytic tumor, rhabdomyosarcoma, synovial sarcoma and undifferentiated pleomorphic sarcoma, cardiac cancer (including sarcoma such as, e.g., angiosarcoma, fibrosarcoma, rhabdomyosarcoma or liposarcoma, myxoma, rhabdomyoma, fibroma, lipoma and teratoma), bone cancer (including osteogenic sarcoma, osteosarcoma, fibrosarcoma, malignant fibrous histiocytoma, chondrosarcoma, Ewing's sarcoma, malignant lymphoma and reticulum cell sarcoma, multiple myeloma, malignant giant cell tumor chordoma, osteochronfroma, osteocartilaginous exostoses, benign chondroma, chondroblastoma, chondromyxoid fibroma, osteoid osteoma and giant cell tumors), hematologic and lymphoid cancer, blood cancer (including acute myeloid leukemia, chronic myeloid leukemia, acute lymphoblastic leukemia, chronic lymphocytic leukemia, myeloproliferative diseases, multiple myeloma and myelodysplasia syndrome), Hodgkin's disease, non-Hodgkin's lymphoma and hairy cell and lymphoid disorders, and the metastases thereof. The lymphoma may be for example a T cell lymphoma such as anaplastic T cell lymphoma.

The cancer may be a solid or liquid cancer, in particular chosen from any one of the cancer disclosed herein.

In some particular embodiments, the cancer is selected from the group consisting of: Melanoma, Renal Cell Carcinoma, Colorectal Cancer, Hepatocellular Carcinoma, Non-Small Cell Lung Cancer, Malignant Pleural Mesothelioma, Esophageal Cancer, Head and Neck Squamous Cell Carcinoma, Urothelial Carcinoma, Primary Hodgkin Lynphoma, Gastric Cancer, Large B Cell Lymphoma, Cervical Cancer, Merkel Cell Carcinoma, Endometrial Carcinoma, Cutaneous Squamous Cell Carcinoma, Triple Negative Breast Cancer, Breast invasive Carcinoma, Pancreatic Adenocarcinoma, Thymoma, Prostate Adenocarcinoma, Ovarian Serous Cystadenocarcinoma, Thyroid Carcinoma, and Sarcoma.

The invention provides also a method for treating cancer, comprising: administering a therapeutically effective amount of the immunocytokine, polynucleotide, vector, pharmaceutical composition according to the invention to the patient.

The immunocytokine, polynucleotide, vector, pharmaceutical composition of the present invention is generally administered according to known procedures, at dosages and for periods of time effective to induce a therapeutic effect in the individual. The immunocytokine, polynucleotide, vector, pharmaceutical composition may be administered by any convenient route, such as in a non-limiting manner by infusion or bolus injection, by absorption through epithelial or mucocutaneous linings (e.g., oral mucosa, rectal and intestinal mucosa, etc.), inhalation, transdermal application or intratumoral administration. The administration can be systemic, local or systemic combined with local; systemic includes parenteral and oral, and local includes local, loco-regional and intra-tumoral. Systemic administration is preferably parenteral such as subcutaneous (SC), intramuscular (IM), intravascular such as intravenous (IV) or intraarterial; intraperitoneal (IP); intradermal (ID), epidural or else. The parenteral administration is advantageously by injection or perfusion. In some particular embodiments, the administration is parenteral and/or intratumoral, preferably intravascular such as intravenous (IV), intratumoral or IV combined with intratumoral.

By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

In some embodiments, the immunocytokine, polynucleotide, vector, pharmaceutical composition is used for the prevention or treatment of humans.

The immunocytokine, polynucleotide, vector, pharmaceutical composition of the invention is advantageously used in combination with another cancer therapy. In particular, the immunocytokine, polynucleotide, vector, and/or pharmaceutical composition of the invention may be administered in combination with targeted therapy; immunotherapy such as immune checkpoint therapy and immune checkpoint inhibitor, co-stimulatory antibodies, and CAR-T cell therapy; anticancer agents including therapeutic agents (chemotherapy) and vaccines against cancer and/or radiotherapy. The combined therapies may be separate, simultaneous, and/or sequential.

Immune checkpoint therapy such as checkpoint inhibitors include, but are not limited to programmed death-1 (PD-1) inhibitors, programmed death ligand-1 (PD-L1) inhibitors, programmed death ligand-2 (PD-L2) inhibitors, lymphocyte-activation gene 3 (LAG-3) inhibitors, T-cell immunoglobulin and mucin-domain containing protein 3 (TIM-3) inhibitors, T cell immunoreceptor with Ig and ITIM domains (TIGIT) inhibitors, B- and T-lymphocyte attenuator (BTLA) inhibitors, V-domain Ig suppressor of T-cell activation (VISTA) inhibitors, Indoleamine 2,3-dioxygenase (IDO) inhibitors, killer immunoglobulin-like receptors (KIR) inhibitors, KIR2L3 inhibitors, KIR3DL2 inhibitors and carcinoembryonic antigen-related cell adhesion molecule 1 (CEACAM-1) inhibitors. In particular, checkpoint inhibitors include antibodies anti-PD1, anti-PD-L1, anti-CTLA-4, anti-TIM-3, anti-LAG3. Co-stimulatory antibodies deliver positive signals through immune-regulatory receptors including but not limited to ICOS, CD137, CD27, OX-40 and GITR.

Examples of anti-PD1 antibodies include, but are not limited to, nivolumab, cemiplimab (REGN2810 or REGN-2810), tislelizumab (BGB-A317), spartalizumab (PDR001 or PDR-001), ABBV-181, JNJ-63723283, BI 754091, MAG012, TSR-042, AGEN2034, pidilizumab, nivolumab (ONO-4538, BMS-936558, MDX1106, GTPL7335 or Opdivo), pembrolizumab (MK-3475, MK03475, lambrolizumab, SCH-900475 or Keytruda) and antibodies described in International patent applications WO2004004771, WO2004056875, WO2006121168, WO2008156712, WO2009014708, WO2009114335, WO2013043569 and WO2014047350.

Examples of anti-PD-L1 antibodies include, but are not limited to, LY3300054, atezolizumab, durvalumab and avelumab.

Example of anti-VISTA antibodies are described in US patent application US20130177557.

Example of inhibitors of the LAG3 receptor are described in US patent US5,773,578.

Example of KIR inhibitor is IPH4102 targeting KIR3DL2.

Targeted therapy are drugs designed to interfere with specific molecules necessary for tumor growth and progression. For example, therapeutic monoclonal antibodies target specific antigens found on the cell surface, such as transmembrane receptors or extracellular growth factors. In some cases, monoclonal antibodies are conjugated to radio-isotopes or toxins to allow specific delivery of these cytotoxic agents to the intended cancer cell target. Small molecules can penetrate the cell membrane to interact with targets inside a cell. Small molecules are usually designed to interfere with the enzymatic activity of the target protein such as for example proteasome inhibitor, tyrosine kinase or cyclin-dependent kinase inhibitor, histone deacetylase inhibitor. Targeted therapy may also use cytokines. Examples of such targeted therapy include with no limitations: Ado-trastuzumab emtansine (HER2), Afatinib (EGFR (HER1/ERBB1), HER2), Aldesleukin (Proleukin), alectinib (ALK), Alemtuzumab (CD52), axitinib (kit, PDGFRbeta, VEGFR1/2/3), Belimumab (BAFF), Belinostat (HDAC), Bevacizumab (VEGF ligand), Blinatumomab (CD19/CD3), bortezomib (proteasome), Brentuximab vedotin (CD30), bosutinib (ABL), brigatinib (ALK), cabozantinib (FLT3, KIT, MET, RET, VEGFR2), Canakinumab (IL-1 beta), carfilzomib (proteasome), ceritinib (ALK), Cetuximab (EGFR), cofimetinib (MEK), Crizotinib (ALK, MET, ROS1), Dabrafenib (BRAF), Daratumumab (CD38), Dasatinib (ABL), Denosumab (RANKL), Dinutuximab (B4GALNT1 (GD2)), Elotuzumab (SLAMF7), Enasidenib (IDH2), Erlotinib (EGFR), Everolimus (mTOR), Gefitinib (EGFR), Ibritumomab tiuxetan (CD20), Sonidegib (Smoothened), Sipuleucel-T, Siltuximab (IL-6), Sorafenib (VEGFR, PDGFR, KIT, RAF),(Tocilizumab (IL-6R), Temsirolimus (mTOR), Tofacitinib (JAK3), Trametinib (MEK), Tositumomab (CD20), Trastuzumab (HER2), Vandetanib (EGFR), Vemurafenib (BRAF), Venetoclax (BCL2), Vismodegib (PTCH, Smoothened), Vorinostat (HDAC), Ziv-aflibercept (PIGF, VEGFA/B).

In some embodiments, the immunocytokine, polynucleotide, vector and/or pharmaceutical composition of the invention is administered to the patient in combination with chemotherapy. As used herein, the term "chemotherapy" has its general meaning in the art and refers to the treatment that consists in administering to the patient a chemotherapeutic agent. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g. , calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; methylhydrazine derivatives including N-methylhydrazine (MIH) and procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; anthracyclines, nitrosoureas, antimetabolites, epipodophylotoxins, enzymes such as L-asparaginase; anthracenediones; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide; and pharmaceutically acceptable salts, acids or derivatives of any of the above.

In some embodiments, the immunocytokine, polynucleotide, vector and/or pharmaceutical composition of the invention is administered to the patient in combination with radiotherapy. Suitable examples of radiation therapies include, but are not limited to external beam radiotherapy (such as superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT), 3-dimensional conformal radiation therapy (3D-CRT) and the like); brachytherapy; unsealed source radiotherapy; tomotherapy; and the like. Gamma rays are another form of photons used in radiotherapy. Gamma rays are produced spontaneously as certain elements (such as radium, uranium, and cobalt 60) release radiation as they decompose, or decay. In some embodiments, radiotherapy may be proton radiotherapy or proton minibeam radiation therapy. Proton radiotherapy is an ultra-precise form of radiotherapy that uses proton beams (Prezado Y, Jouvion G, Guardiola C, Gonzalez W, Juchaux M, Bergs J, Nauraye C, Labiod D, De Marzi L, Pouzoulet F, Patriarca A, Dendale R. Tumor Control in RG2 Glioma-Bearing Rats: A Comparison Between Proton Minibeam Therapy and Standard Proton Therapy. Int J Radiat Oncol Biol Phys. 2019 Jun 1;104(2):266-271. doi: 10.1016/j.ijrobp.2019.01.080; Prezado Y, Jouvion G, Patriarca A, Nauraye C, Guardiola C, Juchaux M, Lamirault C, Labiod D, Jourdain L, Sebrie C, Dendale R, Gonzalez W, Pouzoulet F. Proton minibeam radiation therapy widens the therapeutic index for high-grade gliomas. Sci Rep. 2018 Nov 7;8(1):16479. doi: 10.1038/s41598-018-34796-8). Radiotherapy may also be FLASH radiotherapy (FLASH-RT) or FLASH proton irradiation. FLASH radiotherapy involves the ultra-fast delivery of radiation treatment at dose rates several orders of magnitude greater than those currently in routine clinical practice (ultra-high dose rate) (Favaudon V, Fouillade C, Vozenin MC. The radiotherapy FLASH to save healthy tissues. Med Sci (Paris) 2015 ; 31 : 121-123. DOI: 10.1051/medsci/20153102002); Patriarca A., Fouillade C. M., Martin F., Pouzoulet F., Nauraye C., et al. Experimental set-up for FLASH proton irradiation of small animals using a clinical system. Int J Radiat Oncol Biol Phys, 102 (2018), pp. 619-626. doi: 10.1016/j.ijrobp.2018.06.403. Epub 2018 Jul 11).

In some particular embodiments, the immunocytokine, polynucleotide, vector, and/or pharmaceutical composition according to the invention is administered in combination with Immune checkpoint therapy, in particular PD-1 and/or PDL-1 inhibitors.

The practice of the present invention will employ, unless otherwise indicated, conventional techniques which are within the skill of the art. Such techniques are explained fully in the literature.

The invention will now be exemplified with the following examples, which are not limitative, with reference to the attached drawings in which:

### FIGURE LEGENDS

**Figure 1** **: Tumor-associated Treg cells over express CD25 and CTLA-4 as compared with peripheral Treg cells.**

**A/B:** Single-cell RNA sequencing data in NSCLC (internal data and Guo et al, Nat Med, 2018, 24, 978-985; and C/ in liver cancer (Zheng et al, Cell, 2017, 169, 1342-1356) was used to generate violin-plots representing level of expression of CD25 (IL-2RA) and CTLA-4 transcripts in blood Treg cells and in matched tumor-associated Treg cells. **D/E:** CD25 and CTLA-4 protein expression levels in intra-tumor and peripheral Treg cells, CD4+ T conventional (Tconv) and CD8+ T cells quantified by flow cytometry (n=5 NSCLC patients).

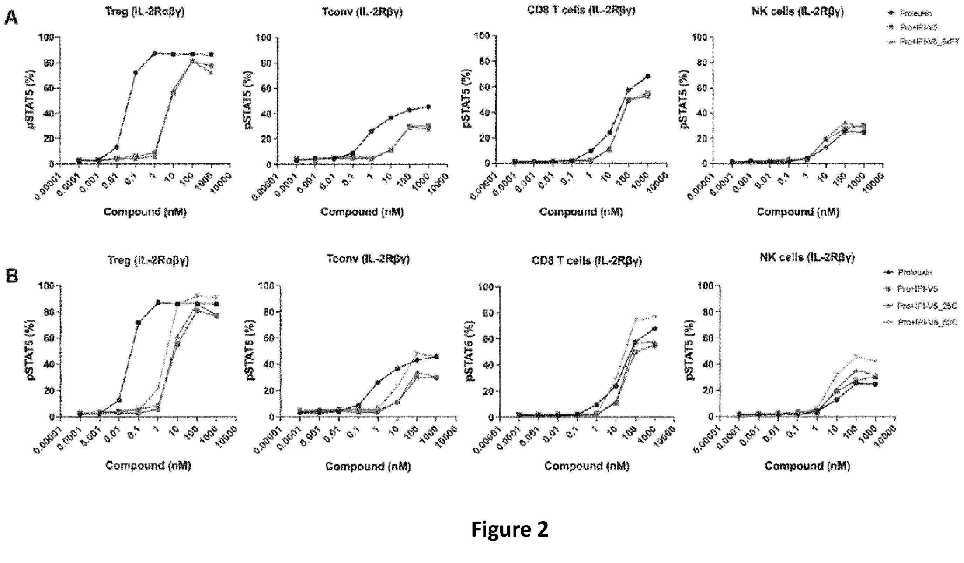

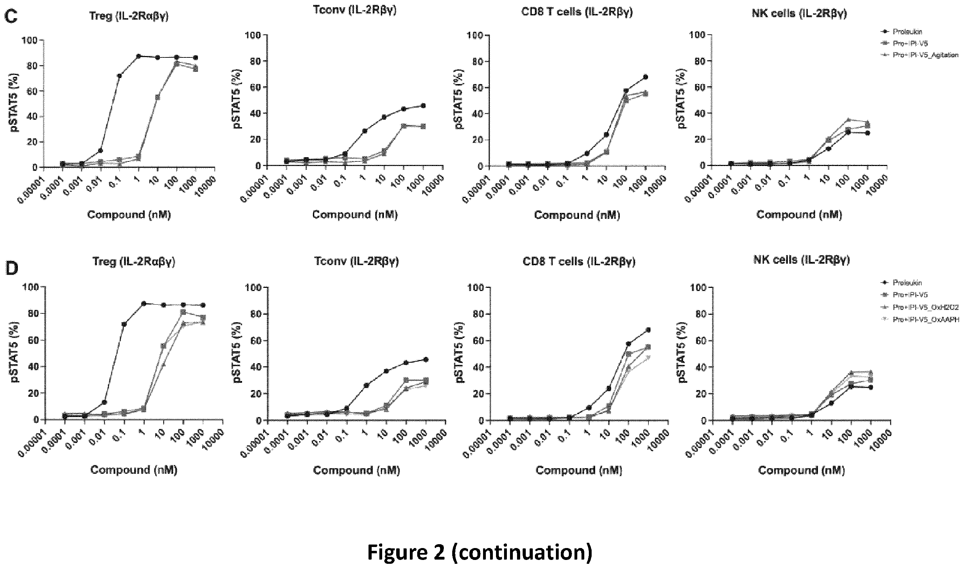

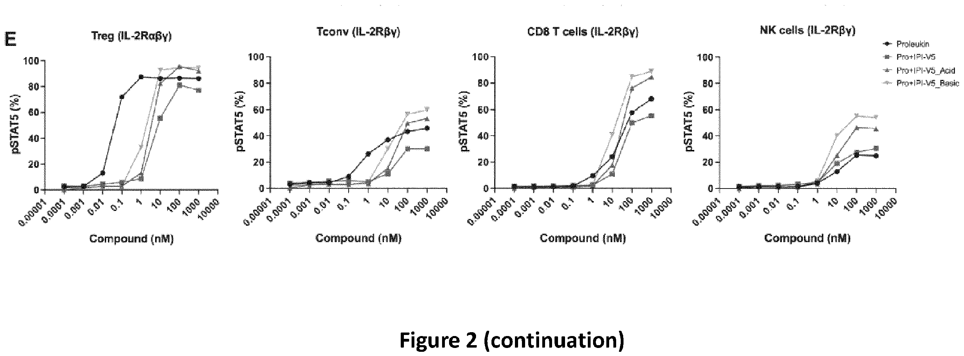

### Figure 2: Stability of IPI-V5 under stressed conditions.

PBMC from healthy volunteers were stimulated for 10 min at room temperature with the indicated concentration of IL-2 (Proleukin^{®}), or with IL-2 + 500 nM IPI-V5 (Pro+IPI-V5), or with IL-2 + 500 nM IPI-V5 that was **A/** subjected to 3 successive freeze-thaw cycles (Pro+IPI-V5_3xFT), **B/** incubated 2 weeks at 25°C (Pro+IPI-V5_25C) or incubated 1 week at 50°C (Pro+IPI-V5_50C), **C/** incubated 48 hours at room temperature under gentle agitation at 300 rpm (Pro+IPI-V5_Agitation), **D/** incubated 3 days at 37°C with 0.01% H₂O₂ (Pro+IPI-V5_OxH2O2) or 1 week at 37°C with 1 mM AAPH (Pro+IPI-V5_OxAAPH) or **E/** incubated 24 hours at room temperature in acidic condition pH 3.5 (Pro+IPI-V5_Acid) or incubated 24 hours at 37°C in basic pH (Pro+IPI-V5_Basic). Cells were fixed, permeabilized and stained with anti-CD3, CD4, CD25, CD56, FoxP3 and pSTAT5 antibodies, and analyzed by flow cytometry. pSTAT5 (%) corresponds to the fraction of all live cells positively stained by the antibody. Data from one representative PBMC donor.

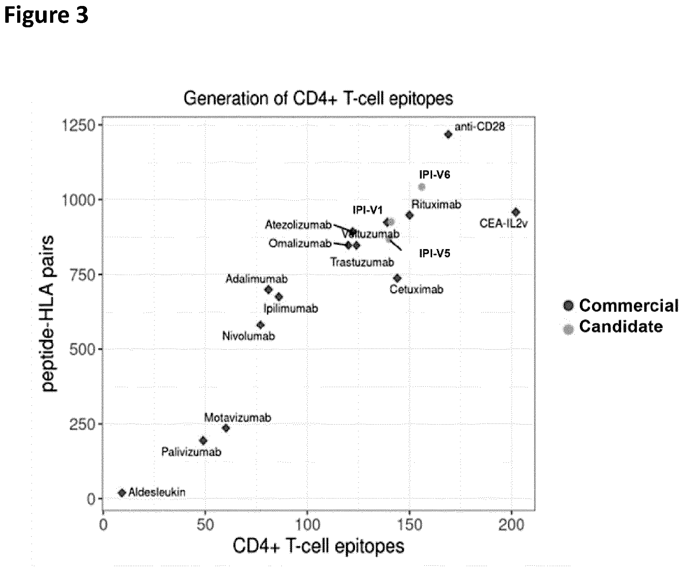

### Figure 3: In silico determination of CD4+ T-cell epitopes derived from immunocytokine candidates, commercial antibodies, and Proleukin.

CD4+ T-cell epitopes (x-axis) were identified using the software netMHCIIpan for Ipi-V1, Ipi, V5 and Ipi-V6 (orange dots), and for commercial and developed therapeutical molecules (green diamonds). Similarly, the number of peptide-HLA-II pairs is depicted (y-axis), which considers the number of HLA-II alleles that all epitopes are predicted to bind.

### Figure 4: Interaction of the different constructs with human FcRn.

In vitro, binding capacity to FcRn was evaluated by ELISA using the LUMIT^{™} FcRn binding Immunoassay kit (Promega), in direct comparison with ipilimumab. Ab control is a strong FcRn binding antibody used as positive control.

### Figure 5: PK profiles in mice after administration of the different compounds.

C57BL6/NRj mice were I.V. injected (retro-orbital injections) with 6mg/kg of IPI-V1, IPI-V5, IPI-V6, or 5mg/kg of ipilimumab; and blood was repeatedly drawn in the tail vein over 72h. Plasma concentrations were evaluated with a non-GLP human IgG ELISA kit (Abcam).

### Figure 6: CTLA-4 Receptor Occupancy.

hCTLA-4-transfected Raji cells were analyzed by flow cytometry to assess the dose-receptor occupancy. Raji cells were incubated for 30 minutes at 4°C. All compounds were revealed with a FITC-labeled secondary antibody against human Fc. Cells were fixed and analyzed with a LSR Fortessa (Becton Dickinson) cytometer. The same analysis on non-transduced Raji cells demonstrated no binding (not shown).

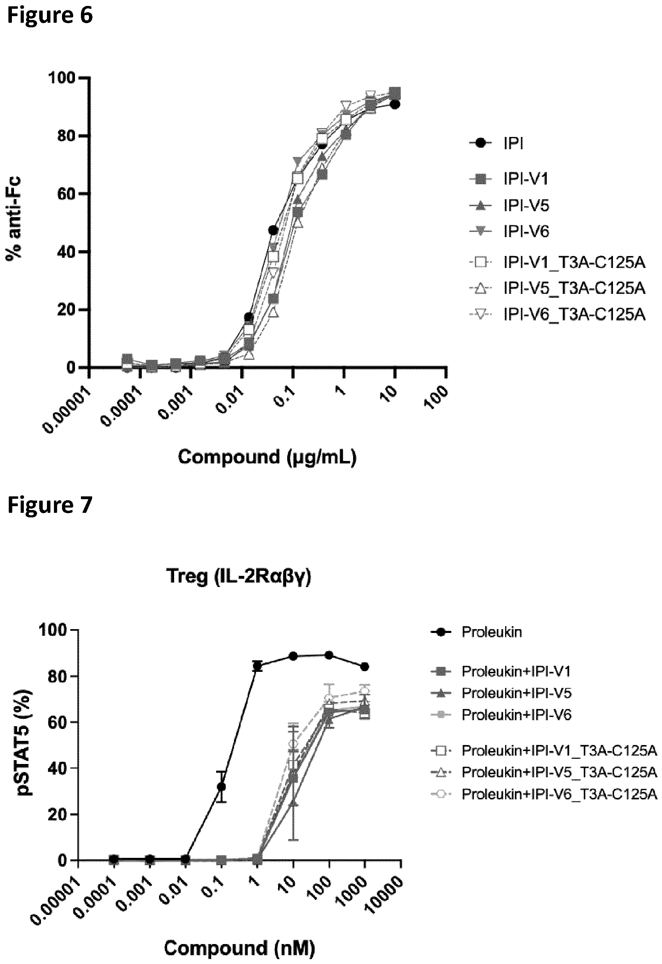

### Figure 7: Inhibition of STAT-5 signaling in Treg cells.

Fresh healthy donor PBMCs were stimulated for 10 min. at room temperature with the indicated concentrations of IL-2 (Proleukin^{®}), or the combination of IL-2 (Proleukin^{®}) and a fixed dose of 500 nM (90 µg/ml) of the indicated compounds. Cells were fixed, permeabilized, and stained with anti-CD3, CD4, CD25, CD56, FoxP3 and pSTAT-5 antibodies, and analyzed by flow cytometry. pSTAT5 (%) corresponds to the fraction of all live cells of each subtype positively stained by the antibody.

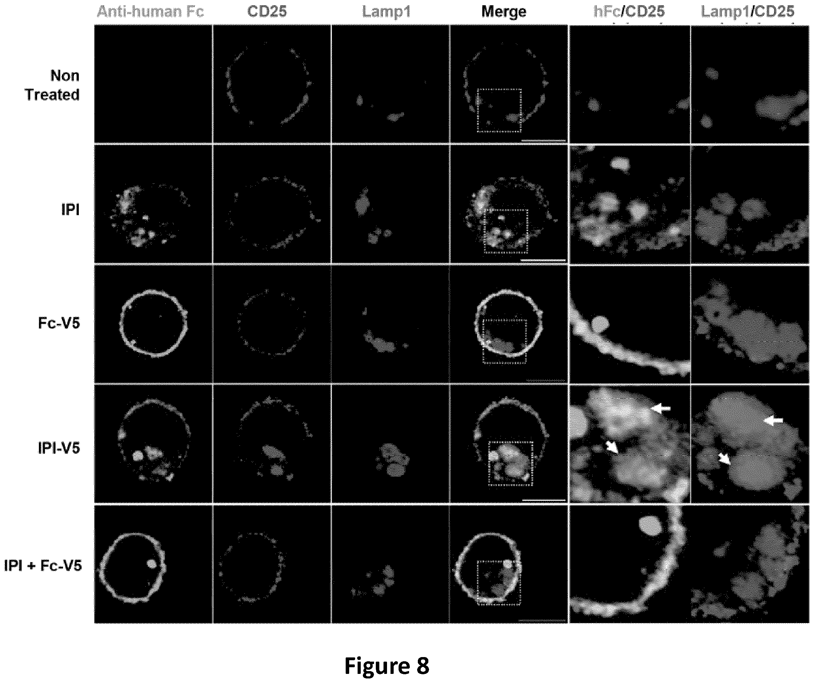

### Figure 8: Immunocytokine-mediated CD25 endocytosis.

In vitro expanded human Treg cells were maintained for 60 min. at 37°C with medium, Fc-V5 (a non-targeted IL-2V5), ipilimumab or IPI-V5. Cells were then fixed, permeabilized, stained with anti-CD25 fluorescent antibodies (red color) or anti-human Fc fluorescent antibodies (green, revealing Fc-V5, ipilimumab or Ipi-V5 localization), and analyzed by confocal fluorescence microscopy. Scale bar: 4µM. Dotted squares show intracellular vesicles. Yellow color means colocalization of red and green colors.

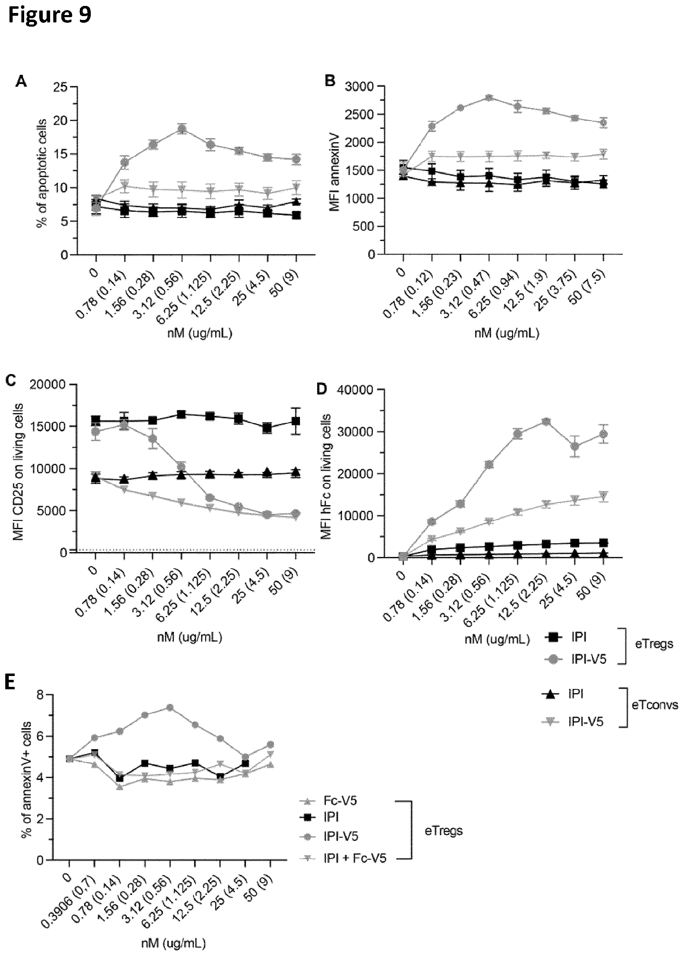

### Figure 9: Immunocytokine preferentially mediates apoptosis of expanded human Tregs.

**A to D.** In vitro expanded human Tregs and Tconvs were cultured at 37°C with cell culture medium containing Ipi-V5 or ipilimumab at different concentrations. After 3 days, apoptosis and Treg markers were evaluated. (A,B) Quantification of the apoptosis by flow cytometry based on annexin V staining. The percentage (A) or MFI (B) of annexin V-positive cells were evaluated among Tregs and Tconv. Treated expanded Tregs and Tconvs were stained for CD25 (C) and human-Fc (D) to assess the behavior of CD25 and Ipi-V5 on the cell surface. Data are presented as mean ± SEM. E. Expanded human Treg cells were maintained in vitro for 3 days with 0.9 ng/ml IL-2 (Proleukin^{®}) and the indicated concentrations of Fc-V5, ipilimumab, Ipi-V5, or Fc-V5 plus ipilimumab. Resulting Treg cells were then analyzed by flow cytometry. % of annexin V+ cells corresponds to the fraction of all gated Treg cells positively stained by the fluorescent annexin V reagent.

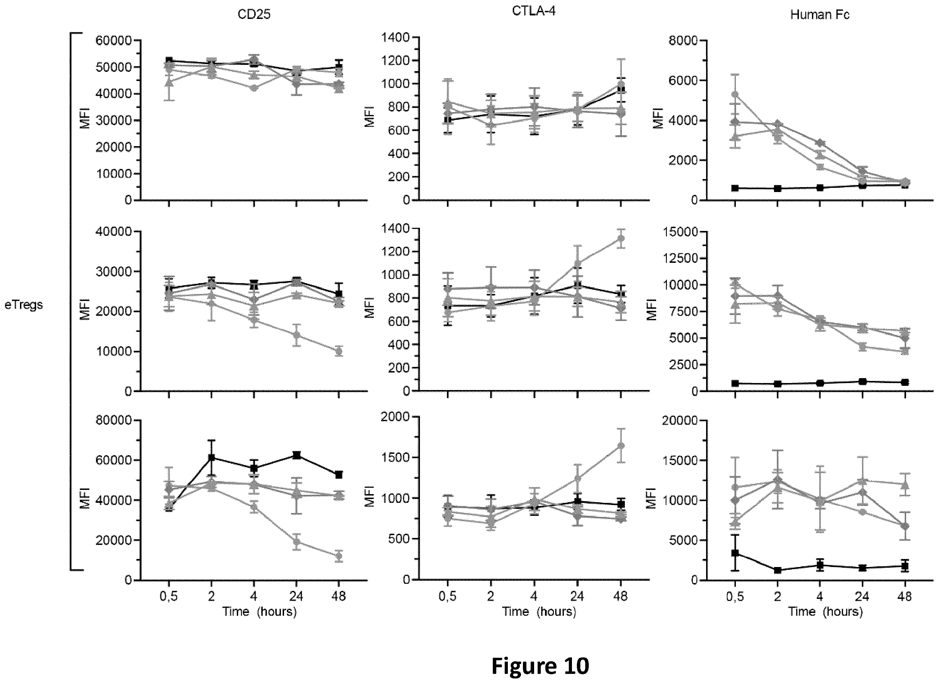

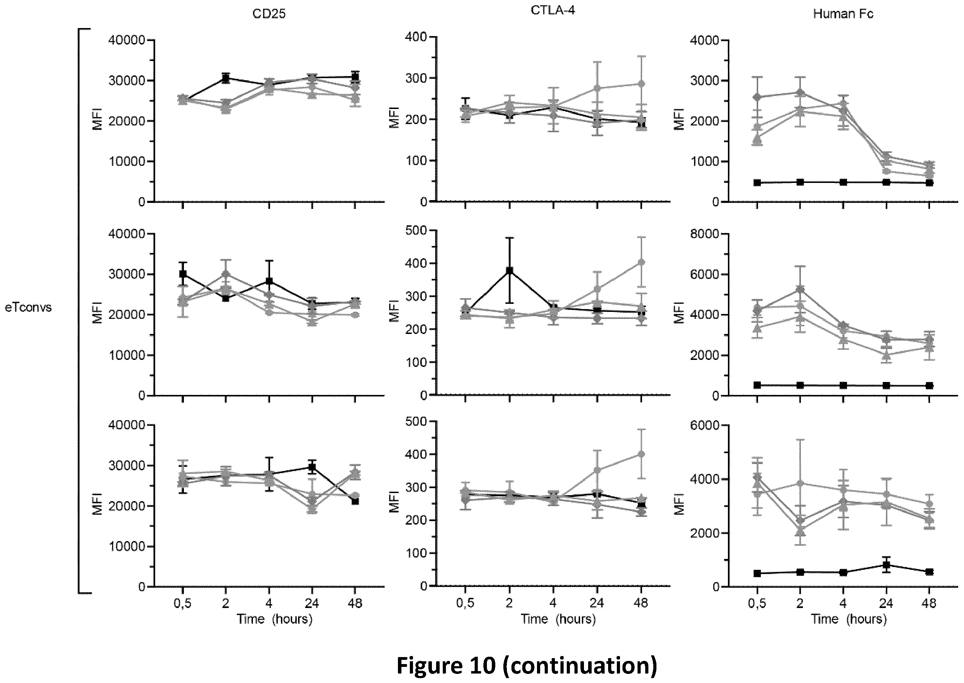

### Figure 10: Immunocytokine induces endocytosis of CD25 in Tregs.

In vitro expanded Tregs and Tconvs were plated and treated with IPI, IPI-V5, Fc-V5 or a combination of IPI+Fc-V5 at three different concentration (0.312, 3.12 and 31.2 nM). Kinetic measurement of endocytosis was performed at different time points. Flow cytometry quantification of the median fluorescent intensity (MFI) of surface expression of CTLA-4, CD25 specific antibodies, and anti-human Fc on Tregs and Tconv was performed (n= 2).

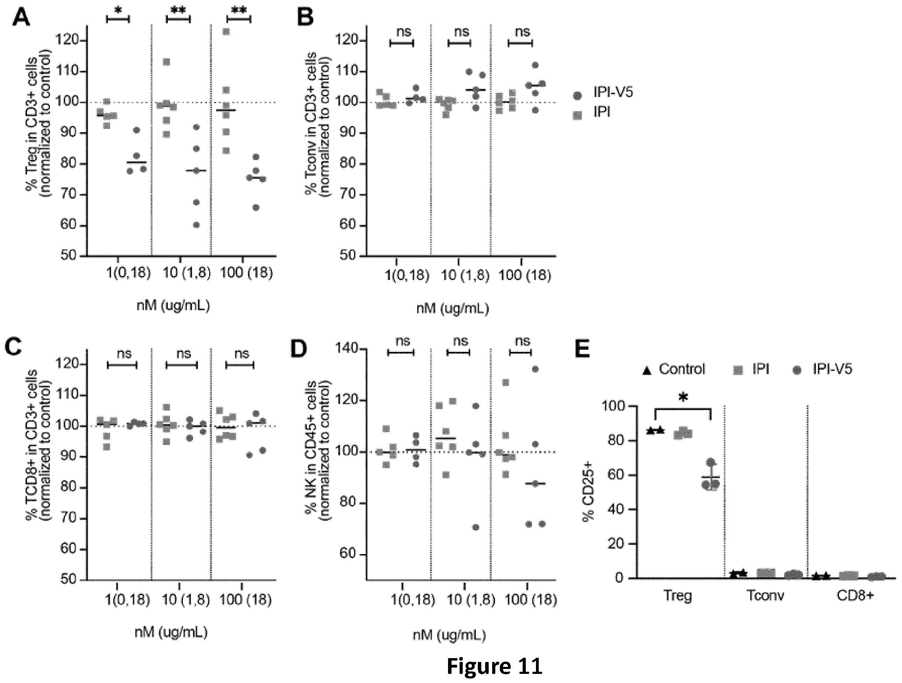

### Figure 11: Treg depletion in breast and lung cancer microenvironment ex-vivo.

Breast and lung cancer samples from 6 patients were dissociated and maintained in vitro in culture medium plus ipilimumab or Ipi-V5. Cells were drawn after 72h and analyzed by flow cytometry on CD45 positive cells. **A:** Treg cells (gated on CD3+ CD4+ FoxP3+); **B**: CD4+ conventional T cells (gated on CD3+ CD4+ FoxP3-), **C:** CD8+ T cells (gated on CD3+ CD8+); **D:** NK cells (gated on CD3-CD56+); **E:** Frequency of CD25+ cells among each cell population. Data shown are individual records from 6 patients/samples analyzed in 3 independent experiments. **, p=0.0066 in non-parametric Mann-Whitney analysis.

### EXAMPLES

### Example 1. Rationale and design of novel immunocytokines

### Material and methods

Raw counts from 3 single cell RNAseq datasets (Tosello & Richer, unpublished; Guo et al, PMID: 29942094, and Zheng et al, PMID: 28622514) were downloaded from Gene Expression Omnibus (GEO). The count matrix from each dataset was fed into Seurat and analyzed independently with standard pipeline. Briefly, "NormalizeData" function with default parameters was applied to normalize the expression level of genes in each single cell for both blood and tumor tissue. Then, 3,000 highly variable genes were identified using the "FindVariableFeatures" functionwith 'vst' method. The "ScaleData" function was used to scale and center gene expression matrices after regressing out heterogeneity associated with mitochondrial contamination. To perform clustering, the dimensionality of the data was determined by calculating relevant principal components using the ElbowPlot function. Relevant principal components were selected to construct the shared nearest neighbor (SNN) graph with "FindNeighbors" function, and clusters were determined using the Louvain algorithm. The uniform manifold approximation and projection (UMAP) was finally applied based on the above described SNN graph to visualize the single cell transcriptional profile in 2D space. Annotation of the clusters was performed using marker genes and published gene signatures. Imputation of missing values in the count matrix was performed using Adaptively thresholded Low-Rank Approximation (ALRA, PMID: 35017482). The expression of CTLA4 and CD25 was depicted using Violin Plots showing ALRA-imputed expression values.

Tumor samples were cut into small fragments using a scalpel and digested with 0.1 mg/ml Liberase TL (Roche) in the presence of 0.1 mg/ml DNase (Roche) for 30 min in CO2-independent medium (Gibco). Cells were then mechanically dissociated with a 2,5mL syringe's plunger on a 40-µm cell strainer (BD) to obtain single cell suspensions. Tumor cell suspensions were washed in PBS and incubated with LIVE/DEAD Fixable Cell Dead Stain (Invitrogen) during 15 min at RT. Next, cells were washed and incubated with fluorochrome labelled Abs for 30 min at 4°C. The following antibodies were used for surface staining: CD3 R718 (clone UCHT1), CD4 BV786 (clone RPA-T4), CD25 BUV737 (clone 2A3), CD8 BUV496 (clone RPA-T8) from BD. For intranuclear staining, cells were fixed, permeabilized and stained with Foxp3/Transcription Factor Staining Buffers (eBioscience). CTLA4 BB700 (clone BNI3) and FOXP3 PE-CF594 (clone 236A/E7) were used for Intracellular staining. Cells were acquired on a Fortessa flow cytometer (BD) and analysed using FlowJo software (V.10). Tregs were identified as CD3+CD4+FOXP3+, Tconvs as CD3+CD4+FOXP3- and CD8 as CD3+CD8+ cells. CD25 and CTLA4 median intensity was calculated for all the populations of interest.

### Results

Treg cell survival is under the dependency of interleukin-2 (IL-2), which is mainly produced by effector T cells and NK cells, and signals in Treg cells via the high affinity IL-2 receptor (IL-2R) complex composed of alpha, beta and gamma chains (respectively CD25, CD122 and CD132). The key data that led to the development of the new immunocytokine is the observed selective higher differential expression levels of CD25 and CTLA-4 transcripts (Figure 1 A-C) and proteins (Figure 1 D,E) by tumor-associated Treg cells, compared to blood Treg cells and to other lymphocyte types. In contrast, in the tumor microenvironment, effector T cells and NK cells are chronically activated and express mainly the intermediate affinity IL-2R composed of the beta and gamma chains. Tumor-associated Treg cells thus differentiate from tumor-associated effector T cells, and from peripheral Treg cells by a higher expression of CD25 (IL2RA), as observed in non-small cell lung cancer (NSCLC) and liver cancer samples (**Figure 1****, A to C,** upper panels and Figure 1D). CD25 overexpression thus allows preferential targeting of tumor-associated Treg cells. Anti-tumor immune responses are also under the control of Cytotoxic T-lymphocyte-associated protein 4 (CTLA-4). While CTLA-4 is constitutively expressed in Treg cells both in and outside cancer tissues, cell surface CTLA-4 is minimally detectable among circulating and lymphoid organs Treg cells (**Figure 1****, A to C,** lower panels and Figure 1 E). In contrast, tumor-associated Treg cells express high levels of CTLA-4 on their surface (Figure 1 E).

Based on these findings, the inventors have developed a novel immunocytokine (ICK) aiming at efficiently and selectively depleting tumor infiltrating Tregs, in particular tumor-associated Treg cells with minimal impact on effector cells (CD8+ T cells, CD4+ Tconv cells, NK cells), by targeting the preferentially expressed CTLA-4 and CD25 membrane proteins. This novel immunocytokine is used to further deprive Tregs of IL-2 by down-regulating CD25, selectively targeting Tumor-associated Treg cells to restore antitumoral responses.

To that end, six different ICKs made of an anti-CTLA-4 antibody fused to different IL-2 muteins have been designed, produced and evaluated for their biologic activity and developability properties. The anti-CTLA-4 antibody was the recombinant humanized IgG1 monoclonal antibody, ipilimumab (US Patent 7,605,238 B2). The IL-2 muteins were Treg-specific IL-2R antagonists previously disclosed in WO 2020/201095 and their derivatives including a 3T to A amino acid substitution (to control for potential O-glycosylation in the T residue) and a 125 C to A substitution (to control for free 125 cysteine that may induce di-sulfide bounds), namely IL-2V1, IL-2V1_T3A-C125A, IL-2V5, IL-2V5_T3A-C125A, IL-2V6, IL-2V6_T3A-C125A. IL-2V1 comprises the substitutions K9E, L12E, H16R, L19R, M23L, N26K, S87N, V91K, E95K, N119K, T123A and S127K; IL-2V5 comprises the substitutions K9E, L12E, H16R, L19R, M23L, N26K, Y31P, S87N, V91K, E95K, N119K, T123A and S127K ; IL-2V6 comprises the substitution K9E, L12E, H16R, L19R, M23L, N26K, K49Q, E52S, R81E, D84N, S87N, V91K, E95K, N119K, T123A, T131R and L132S. (GGGGS)3 linkers are connecting the C-terminal amino acids of the antibody heavy chains with the N-terminal amino acids of the IL-2 muteins. The resulting immunocytokine is a 180 KDa homodimer formed by an anti-CTLA-4 antibody fused to two IL-2 muteins via (GGGGS)3 linkers. The immunocytokines are named indifferently IPI-Vno., where no. is the number of the IL-2 variant, e.g., 1, 5 or 6.

### Example 2. Identification and characterization of immunocytokine

### 1. Manufacturability

### Materials and Methods

Expression vectors SLXplasmid_220_Puro_BT+ and SLXplasmid_221_Hygro+ (Selexis, Plan-Les-Ouates, Swizerland), were used for direct implementation of the heavy and light chains of the immunocytokines (1 light chain and 6 heavy chain variants). Expression vectors contain the bacterial beta-lactamase gene from Transposon Tn3 (AmpR), conferring ampicillin resistance and the bacterial ColE1 origin of replication. As derivatives of pGL3 Control (PromegaAs derivatives of pGL3 Control (Promega, the terminator region of Selexis expression vector bears a SV40 enhancer positioned downstream the BGH polyadenylation signal. Vectors also include a human X_29 SGE downstream the expression cassette and an integrated Puromycin or Hygromycin resistance gene under the control of the SV40 promoter.

Expression vectors encode the Gene of Interest under the control of the hEF-1 alpha promoter coupled to a CMV enhancer. Plasmids were used to transform 50µL of competent DH5 alpha cells (Invitrogen, cat # 18265-017). One bacterial clone was expanded in 150 mL LB+100µg/mL ampicillin in 300 mL Erlenmeyer flask for bulk extraction of plasmid DNA using NucleoBond Xtra Midi Plus EF, Midi kit (Macherey-Nagel), according to the manufacturer's instructions. Before transfection of CHO cells, the plasmid DNA was linearized by digesting 70 µL of the plasmid midi-preparation with Pvul-HF (NEB). The verified digestion mix was electrophoresed on agarose gel and the DNA band containing the mammalian expression cassette for the Gene of Interest was cut out of the gel, purified using standard techniques and quantified bu NanoDrop^{™}One^{C} (Thermo Fisher). Transgene sequences were verified using the Sanger sequencing technique.

These monocistronic vectors (containing the Puromycin and Hygromycin resistance cassettes) were used to generate cell lines by transfecting the gene of interest in the CHO-M cell line by using Microporation, followed by two rounds of cloning to provide high productive clonal cell line. The wild-type CHO-K1 cell line (Lot. 4765275) was derived as a subclone from the parental CHO cell line originally provided by Dr. T. Puck, University of Chicago, USA, and adapted in BalanCD medium supplemented with 6 mM L-Glutamine. Foe cell lines generation, BalanCD medium (Lot. 91128210913) supplemented with 6 mM L-Glutamine (Lot. 7511020) and 1/400 of Penicillin/Streptomycin (Lot. 0000115461) was pre-warmed by plating 1 mL into one well of a 24-well plate (per microporation set) and incubated at 37°C, 5% CO2. A total amount of 3 µg of linearized DNA (monocistronic vectors carrying Puromycin and Hygromycin resistance cassettes, named Puro_BT+_SLX-595X_SP51H_V5_Hc_Sc and Hygro_BT+_SLX-595X_SP51L_Lc_Sc, respectively), was prepared ahead in sterile reaction tubes per microporation set (plus one GFP control). The MicroPorator (Invitrogen NeonTM transfection system) set up was done by filling 3 mL of Electrolyte Buffer E (Lot. 2B17161) into a specific tube (included in the Invitrogen NeonTM transfection system kit MP-100) and with defined pulse conditions for CHO-M cells (1130V, 20 ms and 3 pulses). Cells were prepared shortly before the transfection procedures to avoid reduction of cell viability and transfection efficiency. CHO-M cells were counted and the viability was determined by using Trypan-Blue staining (97.6% viability for seedtrain culture code: S-22-CHOM-2#180-8). The needed amount of cells (3.4x105 cells) was centrifuged (400 g, 5 min, room temperature). The cell pellets were gently resuspended in Resuspension Buffer R (Lot. 2B17161) to a cell concentration of 3.4x107 cells/mL. 10 µL of cell suspension were immediately transferred into the DNA tubes and mixed very carefully. Cell-DNA mixture was aspirated by the specific MicroPorator pipette and placed into the pipette station. After the microporation, cells were transferred into the previously prepared 24-well plate and incubated overnight in a static, humidified incubator at 37°C and 5% CO2. Transfection efficiency was controlled by using a GFP expressing vector in parallel (microscopic inspection conducted the following day showed normal transfection efficiency between 50-70%).

Two days after transfection, part of the transfected cells of each pool was transferred into one omnitray plate at the concentration of 5'000 cells/mL in semi-solid medium (WFI (Lot. L201015 and L210117), CloneMedia CHO Growth A (Lot. 99901210801) supplemented with 6 mM L-Glutamine (Lot. AG29701757 and 7511020), CloneDetect (Lot. CDU0520A), 3 µg/mL of Puromycin (Lot. 039M4114V) and 250 µg/mL of Hygromycin (Lot. HGG-41-06)). Ten days later, plated cells were screened by ClonePix2 and candidates were picked and transferred into 96-well plates (BalanCD medium (Lot. 91128210913), supplemented with 6 mM L-Glutamine (Lot. 7511020), 1/100 of Penicillin/Streptomycin (Lot. 0000115461), 5 µg/mL of Puromycin (Lot. 039M4114V) and 500 µg/mL of Hygromycin (Lot. HGG-41-06)). Following the transfer of all candidates into 96 deep-well plates, cells were screened in a 96 deep-well fed-batch.

### Results

12 candidates of product (6 variants, 2 transfection ratios) were produced, purified and analyzed first in Ambr15 and then in 1L shake flask. Transient transfection (Ratio LC/HC 1: 3) results are not shown as the productivity was not satisfactory. Transient transfection (Ratio LC/HC 1: 1.3) results are described below. Ambr15 Fed-batches have been produced to evaluate the production yield, monomericity post protein A purification and purity on nonreduced and reduced LabChip capillary SDS-Page.

**Table 1: Small scale production of immunocytokines**

| | **Ambr15 14 day fed-batch production** | | | |
|---|---|---|---|---|
| Variant | PA-HPLC Titer (1:1.3 HC:LC ratio only) | % HMW/Mono/LMW (SEC) post-protein A | % HMW/Main/LMW CE-SDS (NR) | % HC/LC/Other CE-SDS (R) |
| Ipi-V1 | 0.99 g/L (Bdp) | 2.42/91.26/6.32 | 0/92.19/7.81 | 79.62/19.22/1.16 |
| Ipi-V1 T3AC125A | 1.33 g/L (Ddp) | 14.70/76.64/8.66 | 0/87.11/12.89 | 73.83/19.71/6.46 |
| Ipi-V5 | 1.46 g/L (Hdp) | 0.83/93.37/5.80 | 0/93.00/7.00 | 80.53/19.17/0.30 |
| Ipi- V5 T3A C125A | 1.39 g/L (Kdp) | 4.05/91.59/4.36 | 0/92.48/7.52 | 78.45/19.66/1.89 |
| | 1.15 g/L (Kcp) | 2.5/96.9/0.60 | 0/90.62/9.38 | 77.91/20.19/1.43 |
| Ipi-V6 | 0.69 g/L (Pdp) | 12.49/86.13/1.38 | 0/90.33/9.67 | 78.04/19.51/2.45 |
| | 1.04 g/L (Pcp) | 14.00/85.40/0.70 | 0/90.61/9.39 | 78.10/20.16/1.31 |
| Ipi-V6 T3A C125A | 0.80 g/L (Udp) | 44.5/53.3/2.2 | 0/70.67/29.33 | 68.48/19.82/11.70 |
| | 1.08 g/L (Ucp) | 21.3/77.6/1.1 | 0/76.69/23.31 | 69.50/19.93/10.27 |

Productivity data are all in favor of variant V5, with a better production yield, less fragmentation and less aggregates. No clear difference can be highlighted between V5 and V5 (mutated) variants. However, the T3A-C125A double mutation looked detrimental for productivity in V1 and V6 variants.

Twelve One-Liter Pools have then been produced and purified on Protein A capture chromatography to evaluate their manufacturability. Results are presented below.

### Protein A capture

| | |
|---|---|
| Column: | HiTrap MabSelect PrismA 5 mL. |
| Applied to column: | About 1 L of SN |
| Buffer A: | PBS, pH 7.4 |
| Buffer Bi: | 0.1 M Na-citrate, pH 3.5 |
| Buffer B2: | 0.1 M Glycine-HCI pH 2.7 |
| Flow rate: | 1.0 mYmin |
| Wash: | 5 CV PBS, 850 mM NaCI |
| Elution: 1 | 00% B1 -> 100% B2 |
| Neutralization: | to pH 5-6 with 1.5 M Tris pH 8.8 |

**Table 2: Large scale production of immunocytokines**

| Variant | 1L shake flask 10 day feed-batch production | | | | | | |
|---|---|---|---|---|---|---|---|
| | Titer (1:1.3 HC:LC ratio only) | % recovery post-protA* | % HMW/Mono/LMW (SEC) post-protA* | % HMW/Mono/LM W (pSEC) pre-prepSEC (approx)* | % HMW/Mono/ LMW (SEC) post-prepSEC* | % HMW/Main/LM W CE-SDS(NR) post-prepSEC* | % HC/LC/other CE-SDS(R) post-prepSEC* |
| Ipi- V1 | 0.37 g/L (Bdp) | 74 | 1.48/98.52/0 | 5/95/0 | 0/100/0 | 0/94.0/6.0 | 81.5/17.9/0.6 |
| Ipi- V1 T3A C125A | 0.20 g/L (Ddp) | 68 | 45.91/49.24/4.85 | 60/40/0 | 0/100/0 | 0/47.6/52.4 | 64.0/16.8/19.3 |
| Ipi- V5 | 0.58 g/L (Hdp) | 83 | 0/100/0 | 2.5/97.5/0 | 0/100/0 | 0/94.7/5.3 | 81.1/17.7/1.2 |
| Ipi-V5 T3A C125A | 0.61 g/L (Kdp) | 64 | 3.47/96.53/0 | 10/90/0 | 0/100/0 | 0/93.7/6.3 | 79.9/18.6/1.5 |
| Ipi- V6 | 0.20 g/L (Pdp) | 100 | 33.53/66.47/0 | 36/64/0 | 0/100/0 | 0/94.2/5.8 | 80.3/17.6/2.1 |
| Ipi- V6 T3A C125A | 0.22 g/L (Udp) | 59 | 79.29/20.71/0 | 76/24/0 | 0/100/0 | 0/73.3/26.7 | 69.9/11.7/18.5 |

Results are consistent with what was observed in small-scale with productivity data all in favor of variant V5, with a better production yield, less fragmentation and less aggregates. No clear difference can be highlighted between V5 and V5 (mutated) variants. T3A-C125A double mutation looked detrimental for productivity in V1 and V6 variants. Protein A titers from CHO pools demonstrated the best productivity and best product profile with V5 candidate. Ipi-V5 (with or without the two mutations) seemed to present better profiles. Moreover, for Ipi-V1 and Ipi-V6, the variants presenting the mutations looked detrimental for productivity (more aggregates, more fragments).

Altogether, these production data indicated that IPI-V5 was better produced and presented a more homogenous biochemical profile than the other ICK candidates bearing different IL-2V.

### 2. Stability studies under stressed conditions

All 6 ICK candidates were subjected to a stability study after applying different stress conditions (freeze/thaw, temperature, agitation, pH, oxidation). Subsequent biochemical profiling including monomericity were performed. Data indicated that the selected candidate, IPI-V5, was stable across the different conditions. Later, the biologic activity of IPI-V5 after stress testing was evaluated using a STAT-5 phosphorylation competition assay (**Figure 2**)**.** Briefly, fresh healthy donor PBMCs were stimulated for 10 min. at room temperature with the indicated concentrations of IL-2 (Proleukin^{®}), or the combination of IL-2 (Proleukin^{®}) and a fixed dose of 500nM (90µg/ml) of Ipi-V5. Cells were fixed, permeabilized, and stained with anti-CD3, CD4, CD25, CD56, FoxP3 and pSTAT-5 antibodies, and analyzed by flow cytometry. pSTAT5 (%) corresponds to the fraction of all live cells of each subtype positively stained by the antibody.

The following conditions were tested:
- IPI-V5, 3 cycles of freeze/thaw => 75±10°C (at least 30 min)/RT
- IPI-V5, 2 weeks at 25°C
- IPI-V5, 1 week at 50°C
- IPI-V5, agitation 48h at 300rpm and at RT
- IPI-V5, oxidation stress with 0.01% H₂0₂ at 37°C for 3 days
- IPI-V5, oxidation Stress with 1mM AAPH at 37°C for 1 week
- IPI-V5, acidic pH stress at pH 3.5 (RT) for 24hrs
- IPI-V5, basic pH stress (37°C) for 24hrs

To summarize the results on the IPI-V5 activity of inhibition of WT IL-2-induced STAT-5 phosphorylation: IPI-V5 is stable after 3 Freeze/Thaw cycles, 2 weeks at 25°C and 48 hours of agitation. The impact of the oxidation stress with H₂0₂ is unclear.

### 3. In silico immunogenicity study

### Materials and methods

HLA-II alleles were retrieved at Allele Frequency Net Database (Gonzalez-Galarza, F. F. et al., Nucleic Acids Res., 2020, 48, D783-D788) from HLA-II associated with blood cord and/or Anthropological studies. HLA alleles with elevated frequencies in the Caucasian population were selected for the binding predictions. Binding to the HLA-II molecules was predicted using netMHCIIpan version 4 (Reynisson, B. et al., Nucleic Acids Res., 2020, 48, W449-W454) for peptides of 15 amino acids (15mers) derived rom the immunocytokine candidate molecules and antibodies and cytokines with publicly available sequences. Self peptides from the queried proteins that are present in the Human Proteome GRCh38 or in any of the 29 immunoglobulin germline genes were excluded. A standard quantitative estimate of peptide binding was used as threshold to filter strong (percentile of binding affinity within the top 2% as compared to a pool of random natural peptides), weak (percentile of binding affinity between the top 2% and 10% percentile) and remove non-binder peptides (percentile of binding affinity below the 10%). All coumpounds were characterized by the total number of peptides binding to any HLA-II allotype, the number of unique peptide-HLA pairs and an immunogenicity score that multiplies the binding strength (Score_EL from netMHCIIpan) and median HLA-II allelic frequency.

### Results

Immunogenicity has been evaluated by computational interference of presence of potential CD4+ T-cell epitope sequences presented by all MHC Class II. The outcome indicated that IPI-V5 presents an overall immunogenicity potential (number of potential epitopes and numbers of potential peptide-HLA pairs) falling in the same range as antibodies that are currently in clinical use, such as atezolizumab, trastuzumab, omalizumab, or rituximab; and lower immunogenicity than the IPI-V1 and IPI-V6 versions (**Figure 3**)**.**

### 4. Pharmacokinetic studies

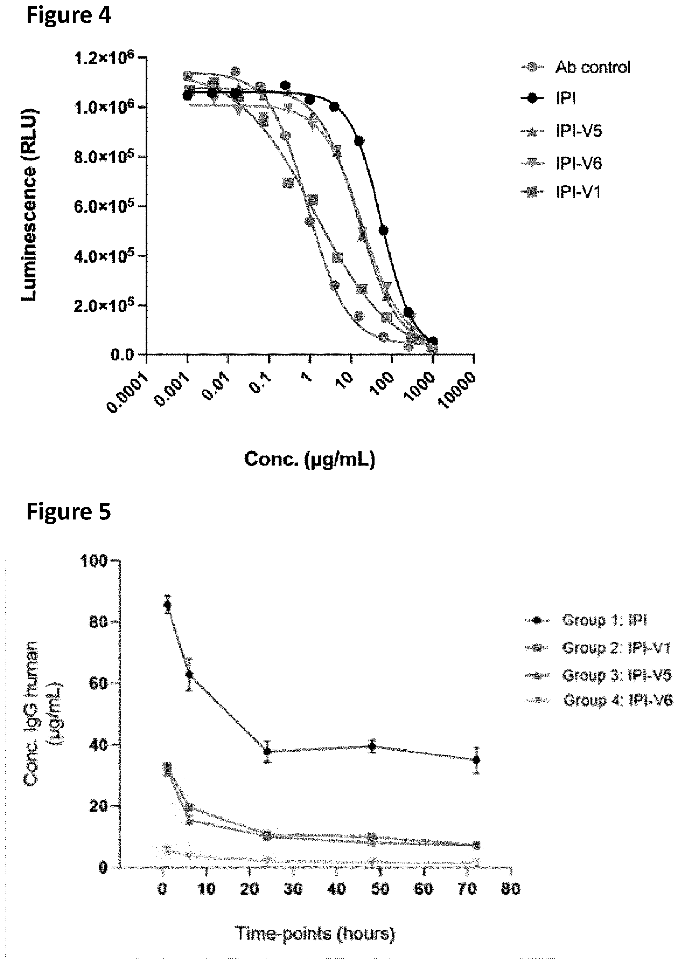

Surrogate PK assessment has been assessed by comparative binding of anti-CTLA-4 antibody Ipilimumab and the different IPI-IL-2 variants to the neonatal Fc receptor (FcRn). The data revealed Ipi-Vx variants presented a FcRn binding equivalent or superior to the comparator ipilimumab, predicting a Fc-mediated extended half-life (**Figure 4**).

Then, mice were injected I.V. (1 injection at Day 0, 6mg/kg) with IPI-V1, IPI-V5, IPI-V6 or ipilimumab and blood was repeatedly drawn in the tail vein over 72h. IPI-variants and ipilimumab were assessed by ELISA in the blood (**Figure 5**).

For IPI-V5, the data indicated a Cₘₐₓ of 32 µg/ml, which is approx. 40% of the calculated theoretical value if a plasma distribution-only is considered (based on an average blood volume of 78 ml/Kg). This indicated a drug disposition, presumably in targets present in the blood and blood-related compartments (lymphoid organs). Indeed, if Ipi-V5 does not compete out murine IL-2, it nevertheless binds to murine IL-2R. After this initial distribution/retention period, the terminal elimination half-life (T1/2) of Ipi-V5 was of approx. 72h, which is in the range of what is expected from a human monoclonal antibody in mice. In contrast, ipilimumab distributed in the expected blood volume, resulting in a Cₘₐₓ of approx. 85 µg/ml, owing to the absence of interaction with mouse CTLA-4. The T_{1/2} found after I.V. administration of ipilimumab was comparable with Ipi-V5. Although these data must be confirmed in non-human primates, they indicate that the T_{1/2} of Ipi-V5 might be similar to the T1/2 of ipilimumab (which in human is 15.4 days).

### 5. Biological activity

### - Assessment of immunocytokine candidates binding activity to CTLA-4

Receptor occupancy of the different immunocytokine candidates (IPI-V1, IPI-V5, IPI-V6) was evaluated in vitro, with a binding assay on Raji C4 cells expressing human CTLA-4 and no significant differences were observed in the dose-receptor occupancy results **(****Figure 6****).**

### - Competition assay to evaluate the IL-2 antagonist activity of the IL-2V moiety of the immunocytokine candidates

STATS phosphorylation competition assay was performed to determine if the IL-2V part of the immunocytokine candidates (IPI-V1, IPI-V5, IPI-V6) can compete with exogenous IL-2 to induce STAT-5 phosphorylation in human Treg. No significant differences were observed among the tested variants **(****Figure 7****).**

Overall, all candidates behave similarly in both assays (i.e; CTLA-4 binding and IL-2V antagonist activity). No impact of "manufacture-friendly" mutations were observed.

### - Immunocytokine candidates mediate CTLA-4-dependent CD25 endocytosis

A particular mechanism of action of the immunocytokine candidates was discovered, namely, that they mediate CTLA-4-dependent CD25 endocytosis in primary human Treg cells.

CTLA-4 is a membrane receptor known to shuttle from the surface to the cytoplasm, where it can undergo endosomal degradation, or can be re-expressed by receptor recycling or by de-novo expression. This cycle defines an important mechanism of suppression of Treg cells that capture the CTLA-4 ligand CD80 from the surface of antigen-presenting cells and eliminate CD80 by trans-endocytosis and endosomal-mediated degradation (Qureshi et al, Science, 2011, 332, 600-603). Ipi-V5 and anti-CTLA-4 antibody (ipilimumab), binding to CTLA-4, are also susceptible to be endocytosed together with CTLA-4 and degraded. Ipi-V5, however, binds both CTLA-4 and CD25, so that it might drive CD25 endocytosis. To verify whether Ipi-V5 drives CD25 endocytosis, confocal microscopy experiments were performed where Treg cells were incubated with ipilimumab or Ipi-V5 for 60 min. at 37 °C, after which time localization of CD25, Ipi-V5, and ipilimumab were analyzed (**Figure 8****).**

It was observed that CD25, originally present solely at the plasma membrane of Treg cells, was found in the cytoplasm after addition of Ipi-V5, but not after addition of anti-CTLA-4 antibody ipilimumab. Addition of a non-targeted IL-2 mutein control molecule (Fc-V5) harboring two IL-2 muteins, but devoid of anti-CTLA-4 activity, did not modify CD25 localization either. In the cytoplasm, CD25 co-localized with Ipi-V5. These data indicate that Ipi-V5 binds to CTLA-4 and to CD25 on the surface of Treg cells, and that the Ipi-V5/CTLA-4/CD25 complex is subsequently endocytosed. Without being bound by theory, the inventors believe that CD25 endocytosis would be observed with any other immunocytokine according to the invention derived from any anti-CTLA-4 antibody other than ipilimumab, as long as, but not exclusively, the anti-CTLA-4 antibody induces receptor cross-linking and clustering of CTLA-4 molecules via endocytosis; or facilitates the formation of clathrin-coated vesicles and subsequent endocytosis; or triggers a conformational change on the cytoplasmic tail of CTLA-4 that serves as internalization signal by recruiting the endocytic machinery; or the anti-CTLA4 antibody binding disrupt lipid rafts thus enhancing the internalization of the ICK-receptor complex.

This CTLA-4-dependent CD25 endocytosis thus represents an additional mechanism of action ofIpi-V5, diminishing the surface expression of CD25 on Treg cells and presumably blunting Tregs capacity to receive IL-2-mediated survival signals. This particular mechanism of action based on endocytosis and reduction of surface CD25 expression on Tregs, should be key to eliminate Tregs and promote an efficient anti-tumor response.

### - Immunocytokine candidates mediate apoptosis predominantly in human Tregs

An assay was performed to evaluate the capacity of **IPI-V5** to induce apoptosis of human expanded T regulatory cells (eTregs) and expanded T conventional cells (eTconv) after expansion with IL-2. Moreover, to assess the expression of markers on Treg cells, the cells were stained with a panel of antibodies. At day -7, PBMCs obtained from healthy donor fresh blood were subjected to magnetic separation on MACS columns using CD25 coated beads. CD25+ fraction was further stained for CD127 and CD25 to sort at the Beckman Coulter Cytoflex sorter to obtain Tregs (CD4+CD8-CD25+CD127lo). Tconv were purified from the CD25- fraction after CD4 selection. Cells were put in culture under activation conditions using antiCD3/antiCD28 beads for 6 days. One day before the beginning of the assay, beads were removed from Tregs and Tconv and placed in X-vivo medium supplemented with 300 iU/ml of Proleukin for 24h rest. On day 0, beads were added to the cells and treated with anti-CTLA-4 antibody Ipilimumab or Ipi-V5 at different concentrations. After 3 days, cells were stained to evaluate apoptosis (Annexin V) and phenotype (Treg marker).

Upon 3 days of treatment with Ipi-V5, an increased percentage of annexinV positive population **(****Figure 9A****)** as well as an increase in the MFI of annexinV **(****Figure 9B****)** was observed in eTregs compared to eTconvs, suggesting that the induction of apoptosis by Ipi-V5 ocurrs predominantly in the eTregs population. Moreover, the induction of apoptosis was dose dependent. Apoptosis induction by anti-CTLA-4 antibody Ipilimumab was lower and similar between eTregs and eTconvs. Induction of apoptosis in eTregs using Ipilimumab alone, IL-2 mutein (Fc-V5) alone or Ipilimumab in combination with IL-2 mutein (Fc-V5) was lower than with Ipi-V5 **(****Figure 9E****).**

CD25 expression was downregulated in a concentration dependent manner, being more pronounced in eTregs than eTconvs **(****Figure 9C****).** Conversely human Fc, which stains the Fc part of IPI and Ipi-V5, was able to detect a more stable presence of the compound and indirectly of CTLA-4 on the membrane surface in eTregs treated with Ipi-V5 **(****Figure 9D****).**

### Immunocytokine candidate Endocytosis kinetics

A second assay was performed to evaluate the kinetic of endocytosis of CD25 and CTLA4 once eTregs and eTconvs are treated with Ipi-V5. Briefly, eTregs and eTconvs were plated and treated with IPI, IPI-V5, Fc-V5 or a combination of IPI+Fc-V5 at three different concentrations in the presence of aCD3/aCD28 beads (treatment at -48h, -24h, -4h, -2h, -0.5h). At different timepoints, cells were harvested, stained and analyzed by FACS.

Surface CD25 expression decreases in Tregs upon treatment with Ipi-V5, in contrast to Tconvs and to anti-CTLA-4 antibody Ipilimumab-treated cells, highlighting the mechanism of action of Ipi-V5 which involves endocytosis of CD25 **(****Figure 10****,** left panels). CTLA-4 follows a rapid recycling process at the cell surface. The increase in CTLA-4 surface expressions after treatment with Ipi-V5 suggests that CTLA-4 expression is stabilized at the surface of the cell, in contrast to the other treatment groups (**Figure 10****,** middle panels). Staining with anti-human Fc revealed that ipilimumab is rapidly lost from the membrane, following the recycling pattern of CTLA-4. Ipi-V5 is detected at the cell surface in a dose-dependent manner and its intensity slowly decreases up to 48h, showcasing a lower endocytosis rate than ipilimumab (**Figure 10****,** right panels).

Although Ipi-V5 binds to both activated CD25-expressing eTconv and eTregs, it only induces apoptosis in Tregs, concomitant to a decrease in CD25 surface expression. At the same time, Ipi-V5 stabilizes CTLA-4 at the plasma membrane in both Tregs and Tconvs and has a slower endocytic kinetic compared to anti-CTLA-4 antibody Ipilimumab.

### Ex-vivo capacity of immunocytokine candidates to decrease Treg proportions among TILs from human NSCLC and BC tumor samples

To investigate the activity of immunocytokine candidates in a representative human tumor microenvironment, dissociated fresh breast (n=2) and lung (n=4) human tumor samples were maintained in vitro up to 72h. Tumor samples were sourced from Institut Curie, Paris, after patient consent and agreement of the ethical committee. Dissociated samples were maintained in culture medium alone or with anti-CTLA-4 antibody ipilimumab or Ipi-Vx at different concentrations, and then analyzed by flow cytometry. The data showed no changes in the frequencies of Treg, Tconv, CD8+ T or NK cells at 24 or 48h timepoints (not shown). However, at 72h, there was a significant decrease in the % of Treg cells among total CD3+T cells, with Ipi-Vx doses ranging from 1 nM (0.09 µg/ml) to 100 nM (9 µg/ml) **(****Figure 11****, A).** In contrast, Tconv (CD4+CD25-), CD8+ T cell or NK cell counts were not significantly modified **(****Figure 11****, B-D**)**.** CD25 expression was significantly decreased in Tregs from samples treated with Ipi-Vx while the levels of CD25 in other cell subsets was undetectable **(****Figure 11****, E).** This ex-vivo experiment confirms that Ipi-Vx can selectively deplete Treg cells in a representative human tumor microenvironment and indicates that 3 days are probably required before Treg cells die from IL-2 starvation in the presence of Ipi-Vx.

### 6. Conclusions

In vitro characterization of six immunocytokines, composed of an anti-CTLA4 monoclonal antibody (Ipilimumab or IPI) bound to two identical molecules of IL-2 variants (IL-2V) chosen from IL-2V1, IL-2V1_T3A-C125A, IL-2V5, IL-2V5_T3A-C125A, IL-2V6, and IL-2V6_T3A-C125A, showed that all IPI-IL-2Vs maintain the biological activity of the two component moieties (i.e. the anti CTLA-4 monoclonal antibody and the IL-2V molecules) in terms of binding to CTLA-4 and Treg-specific IL-2R antagonist activity. However, IPI-V5 showed superior pre-CMC performance (higher production titers, higher purity yields and higher stability), combined with a lower immunogenicity (in silico) and best pharmacokinetic parameters (in mice and in vitro).

Of note, the immunocytokines are able to induce CD25 endocytosis, a property which is absent from the previously disclosed IL-2 variants and provides an additional mechanism to interfere with CD25-mediated survival signals in Tregs. As a result, the immunocytokines are able to kill Tregs selectively and with high efficiency, compared to anti-CTLA-4 antibody or IL-2 mutein used alone or in combination. Ex-vivo experiment confirms that the immunocytokines can selectively deplete Treg cells in a representative human tumor microenvironment. This specific combination of antibody and IL-2 mutein in an immunocytokine thus provides key properties to eliminate tumor-associated Tregs and promote an efficient anti-tumor response. Among, the immunocytokines tested, IPI-V5 stands up as a particularly promising candidate for clinical development of new drug for cancer treatment.

### Description of the Sequences

**SEQ ID NO: 1 Human IL-2**
**SEQ ID NO: 2 ipilimumab HCDR1** GFTFSSYTMH
**SEQ ID NO: 3 ipilimumab HCDR2** FISYDGNNKYYADSVKG
**SEQ ID NO: 4 ipilimumab HCDR3** ARTGWLGPFDY
**SEQ ID NO: 5 ipilimumab LCDR1** RASQSVGSSYLA
**SEQ ID NO: 6 ipilimumab LCDR2** GAFSRAT
**SEQ ID NO: 7 ipilimumab LCDR3** QQYGSSPWT
**SEQ ID NO: 8 ipilimumab VH** 
QVQLVESGGGVVQPGRSLRLSCAASGFTFSSYTMHWVRQAPGKGLEWVTFISYDGNNKYYA
DSVKGRFTISRDNSKNTLYLQMNSLRAEDTAIYYCARTGWLGPFDYWGQGTLVTVSS
**SEQ ID NO: 9 ipilimumab VL** 
EIVLTQSPGTLSLSPGERATLSCRASQSVGSSYLAWYQQKPGQAPRLLIYGAFSRATGIPD
RFSGSGSGTDFTLTISRLEPEDFAVYYCQQYGSSPWTFGQGTKVEIK
**SEQ ID NO: 10 linker** GGGGGSGGGGSGGGGS
**SEQ ID NO: 11 IPI-V5 heavy chain** 
QVQLVESGGGVVQPGRSLRLSCAASGFTFSSYTMHWVRQAPGKGLEWVTFISYDGNNKYYA
DSVKGRFTISRDNSKNTLYLQMNSLRAEDTAIYYCARTGWLGPFDYWGQGTLVTVSSASTK
GPSVFPLAPSSKSTSGGTAALGCLVKDYFPEPVTVSWNSGALTSGVHTFPAVLQSSGLYSL
SSVVTVPSSSLGTQTYICNVNHKPSNTKVDKRVEPKSCDKTHTCPPCPAPELLGGPSVFLF
PPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSC
SVMHEALHNHYTQKSLSLSPGGGGGSGGGGSGGGGSAPTSSTKETQEQLERLLRDLQLILK
GINNPKNPKLTRMLTFKFYMPKKATELKHLQCLEEELKPLEEVLNLAQSKNFHLRPRDLIN
NINKIVLKLKGSETTFMCEYADETATIVEFLKRWIAFCQKIISTLT
**SEQ ID NO: 12 IPI-V5 light chain** 
EIVLTQSPGTLSLSPGERATLSCRASQSVGSSYLAWYQQKPGQAPRLLIYGAFSRATGIPD
RFSGSGSGTDFTLTISRLEPEDFAVYYCQQYGSSPWTFGQGTKVEIKRTVAAPSVFIFPPS
DEQLKSGTASVVCLLNNFYPREAKVQWKVDNALQSGNSQESVTEQDSKDSTYSLSSTLTLS
KADYEKHKVYACEVTHQGLSSPVTKSFNRGEC
**SEQ ID NO: 13 IL-2V1** 
APTSSTKETQEQLERLLRDLQLILKGINNYKNPKLTRMLTFKFYMPKKATELKHLQCLEEE
LKPLEEVLNLAQSKNFHLRPRDLINNINKIVLKLKGSETTFMCEYADETATIVEFLKRWIA
FCQKIISTLT
**SEQ ID NO: 14 IL-2V5** 
APTSSTKETQEQLERLLRDLQLILKGINNPKNPKLTRMLTFKFYMPKKATELKHLQCLEEE
LKPLEEVLNLAQSKNFHLRPRDLINNINKIVLKLKGSETTFMCEYADETATIVEFLKRWIA
FCQKIISTLT
**SEQ ID NO: 15 IL-2V6** 
APTSSTKETQEQLERLLRDLQLILKGINNYKNPKLTRMLTFKFYMPKQATSLKHLQCLEEE
LKPLEEVLNLAQSKNFHLEPRNLINNINKIVLKLKGSETTFMCEYADETATIVEFLKRWIA
FCQSIISRST
**SEQ ID NO: 16 IPI-V5 Heavy chain coding sequence** 
ATGGAGTTCGGACTGTCCTGGGTATTCCTCGTGGCGATCATCAAGGGCGTCCAGTGCCAAG
TGCAGCTGGTGGAGTCCGGTGGCGGAGTCGTACAGCCCGGTCGGTCGCTGCGCCTCTCGTG
CGCAGCGAGCGGGTTCACCTTCTCGTCCTACACCATGCACTGGGTACGTCAGGCACCGGGC
AAGGGCCTGGAGTGGGTCACGTTCATCAGCTATGACGGCAACAACAAGTACTACGCCGACT
CGGTCAAGGGCAGGTTCACCATCTCGCGTGACAACTCCAAGAACACCCTCTACCTCCAGAT
GAACTCCCTCCGCGCAGAGGACACCGCGATCTACTACTGCGCGCGAACCGGTTGGCTGGGC
CCGTTCGACTACTGGGGCCAGGGCACGCTCGTGACCGTTTCGAGCGCCTCCACCAAGGGCC
CGAGCGTCTTCCCCCTGGCGCCAAGCAGCAAGAGCACCAGCGGAGGAACGGCTGCCCTGGG
CTGCCTGGTGAAGGACTACTTCCCGGAGCCGGTCACGGTATCGTGGAACAGTGGCGCGCTG
ACGTCCGGCGTCCACACCTTCCCCGCGGTGCTCCAGTCGAGCGGCCTGTACTCGCTGTCGT
CGGTAGTCACGGTCCCCTCGTCCTCGCTCGGCACGCAGACCTACATCTGCAACGTGAATCA
CAAGCCGTCCAACACGAAGGTGGACAAGCGCGTCGAGCCCAAGTCCTGCGACAAGACGCAC
ACTTGCCCGCCCTGCCCTGCCCCGGAGCTCCTTGGCGGTCCGTCCGTCTTCCTGTTCCCCC
CGAAGCCGAAGGACACCCTGATGATCTCCCGCACCCCGGAGGTGACCTGCGTCGTGGTGGA
CGTGTCCCACGAGGACCCTGAGGTGAAATTCAACTGGTACGTGGACGGCGTCGAAGTCCAC
AACGCCAAGACCAAGCCGCGTGAGGAGCAGTACAATTCCACCTACCGCGTGGTGTCCGTGC
TGACGGTGCTGCACCAGGACTGGCTGAACGGCAAGGAGTACAAGTGCAAGGTGTCCAACAA
AGCCCTCCCGGCTCCCATCGAGAAGACCATCTCCAAGGCCAAGGGCCAGCCCCGTGAACCC
CAGGTCTACACCCTCCCCCCCTCGCGCGATGAGCTGACGAAGAACCAGGTGAGCCTGACCT
GCCTCGTCAAGGGCTTCTATCCGTCCGACATCGCGGTCGAGTGGGAATCGAACGGCCAGCC
CGAGAACAATTACAAGACCACCCCGCCAGTGCTCGACTCCGATGGCAGCTTCTTCCTTTAC
TCGAAGCTCACGGTCGACAAGAGCCGTTGGCAGCAGGGCAACGTGTTCAGCTGCTCGGTCA
TGCACGAGGCCTTGCACAACCACTACACGCAAAAGAGCCTCTCCCTGAGCCCGGGTGGTGG
GGGTGGCTCGGGCGGGGGCGGCAGCGGCGGAGGCGGCTCGGCACCCACCAGTAGCACCAAG
GAAACCCAGGAGCAACTGGAGAGGCTGCTGAGGGACCTGCAGCTGATCCTGAAGGGGATCA
ACAACCCTAAGAACCCGAAGCTGACCCGCATGCTGACCTTCAAGTTCTACATGCCGAAGAA
GGCGACCGAATTGAAGCACCTGCAGTGCCTGGAGGAAGAGCTGAAGCCCCTGGAGGAGGTC
CTCAACCTCGCACAGTCCAAGAACTTCCACCTCCGTCCGCGGGACCTCATCAACAACATCA
ACAAGATCGTCCTCAAGCTCAAAGGCTCGGAAACCACCTTCATGTGCGAGTACGCCGACGA
AACGGCCACCATCGTCGAGTTCCTGAAGCGCTGGATCGCCTTCTGCCAGAAGATCATCTCC
ACGCTGACC
**SEQ ID NO: 17 IPI-V5 Light chain coding sequence**

ATGGACATGCGCGTGCCTGCGCAGCTCCTGGGCCTCCTGCTGTGGCTCAGGGGGGCAC
GCTGCGAGATCGTCTTGACGCAGAGTCCGGGGACCCTCAGCCTGAGCCCCGGAGAGCGGGC
TACCCTGAGCTGTCGTGCGTCGCAGAGCGTCGGCTCGTCCTACTTGGCGTGGTACCAGCAG
AAGCCGGGACAGGCACCGCGCCTCCTGATCTACGGCGCCTTCAGCCGCGCAACGGGCATCC
CCGACCGCTTCTCCGGATCGGGCAGCGGCACCGACTTCACTCTGACGATCTCACGGCTGGA
GCCCGAGGACTTCGCCGTCTACTACTGCCAGCAGTACGGCAGCAGCCCGTGGACCTTCGGC
CAGGGCACCAAGGTGGAGATCAAGCGGACCGTGGCCGCACCGTCGGTCTTCATCTTCCCCC
CGAGCGACGAGCAGCTGAAGTCAGGTACCGCCAGCGTGGTCTGCCTGCTGAATAACTTCTA
CCCCAGGGAGGCGAAGGTGCAGTGGAAGGTCGACAACGCCCTCCAGTCCGGTAACTCCCAG
GAGAGCGTCACCGAGCAGGACTCCAAGGACTCCACCTACTCCCTCTCCTCGACGCTCACCC
TGTCCAAGGCCGACTACGAAAAGCACAAGGTCTACGCGTGCGAGGTGACGCACCAGGGCCT
GTCTTCGCCGGTCACGAAGTCGTTCAACCGGGGTGAGTGC

**Table 3: Anti-CTLA4 antibodies in development (source: TABS antibody database November 14th, 2023)**

| **Name** | **Internal Name** | **Antigen** | **Trials** | **Phase** |
|---|---|---|---|---|
| ipilimumab | Yervoy, MDX-010, MDX101, 10D1, BMS-734016 | CTLA4 | 454 | Approved |
| tremelimumab | ticilimumab, CP-675206, clone 11.2.1 | CTLA4 | 172 | Phase 3 |
| cadonilimab | AK104, cardonilizumab | CTLA4, PD-1 | 38 | Phase 3 |
| erfonrilimab | KN046 | CTLA4, PD-L1 | 10 | Phase 2 |
| zalifrelimab | AGEN1884 | CTLA4 | 9 | Phase 2 |
| quavonlimab | MK-1308 | CTLA4 | 8 | Phase 2 |
| vudalimab | XmAb20717 | CTLA4, PD-1 | 5 | Phase 2 |
| ONC-392 | | CTLA4 | 5 | Phase 3 |
| MEDI5752 | | CTLA4, PD-1 | 5 | Phase 2 |
| botensilimab | AGEN1181 | CTLA4 | 5 | Phase 2 |
| QL1706 | PSB205 | CTLA4, PD-1 | 4 | Phase 2 |
| KD6001 | | CTLA4 | 3 | Phase 1/2 |
| IBI310 | IBI-310 | CTLA4 | 3 | Phase 2 |
| BMS-986218 | | CTLA4 | 2 | Phase 1/2 |
| lorigerlimab | MGD019 | CTLA4, PD-1 | 2 | Phase 1 |
| CS1002 | | CTLA4 | 2 | Phase 1 |
| porustobart | HBM4003, HCAb 4003-2 | CTLA4 | 2 | Phase 1 |
| ADG126 | | CTLA4 | 2 | Phase 1 |
| YH001 | | CTLA4 | 2 | Phase 1/2 |
| ADG116 | | CTLA4 | 2 | Phase 1 |
| ATOR-1015 | ADC-1015 | CTLA4, OX40 | 1 | Phase 1 |
| danvilostomig | SI-B003 | CTLA4, PD-1 | 1 | Phase 1 |
| BMS-986288 | | CTLA4 | 1 | |
| BMS-986249 | | CTLA4 | 1 | Phase 1/2 |
| ADU-1604 | | CTLA4 | 1 | Phase 1 |
| BCD-145 | Q3W | CTLA4 | 1 | Phase 1 |
| REGN4659 | | CTLA4 | 1 | Phase 1 |
| bavunalimab | XmAb22841, pavunalimab | CTLA4, LAG-3 | 1 | Phase 1 |
| KN044 | | CTLA4 | 1 | Phase 1 |
| BT-001 | TG6030 | CTLA4 | 1 | Phase 1/2 |
| XTX101 | | CTLA4 | 1 | Phase 1/2 |
| JK08 | | CTLA4 | 1 | Phase 1/2 |
| TG6050 | | CTLA4 | 1 | Phase 1 |

## Claims

1. A fusion protein comprising an anti-CTLA-4 antibody, and an IL-2 variant which is a regulatory T cell (Treg)-specific IL-2 receptor antagonist comprising the substitutions K9E, L12E, H16R, L19R, M23L, N26K, S87N, V91K, E95K, N119K, T123A; and further comprising the subsitutions: (i) S127K, Y31P and S127K; or (ii) K49Q, E52S, R81E, D84N, T131R and L132S, the indicated positions being determined by alignment with human IL-2 (SEQ ID NO: 1), and wherein the N-terminus of the IL-2 variant is fused to the C-terminus of the antibody heavy chain.

2. The fusion protein according to claim 1, which induces CD25 endocytosis.

3. The fusion protein according to claim 1 or claim 2, which induces Treg apoptosis.

4. The fusion protein according to any one of claims 1 to 3, which depletes selectively tumor-associated Tregs.

5. The fusion protein according to any one of claims 1 to 4, wherein the anti-CTLA-4 antibody comprises a H-CDR1 of SEQ ID NO: 2, H-CDR2 of SEQ ID NO: 3, a H-CDR3 of SEQ ID NO: 4, a L-CDR1 of SEQ ID NO: 5, a L-CDR2 of SEQ ID NO: 6 and a L-CDR3 of SEQ ID NO: 7 or variants further having one or more conservative substitutions on one or more of these CDRs.

6. The fusion protein according to claim 5, wherein the anti-CTLA-4 antibody comprises a VH domain having at least 85 % identity with SEQ ID NO: 8 and a VL domain having at least 85 % identity with SEQ ID NO: 9.

7. The fusion protein according to any one of claim 1 to 6, wherein the anti-CTLA-4 antibody is ipilimumab, tremelimumab, or a functional variant thereof.

8. The fusion protein according to any one of claims 1 to 7, wherein the antibody comprises a human IgG1 Fc domain and/or a human Ig kappa light chain constant domain, or is a nanobody.

9. The fusion protein according to any one of claims 1 to 8, wherein the IL-2 variant has at least 85% sequence identity with any one of SEQ ID NO: 13 to 15.

10. The fusion protein according to any one of claims 1 to 9, which further comprises a linker between the antibody heavy chain C-terminus and the IL-2 variant N-terminus; preferably comprising the sequence SEQ ID NO: 10.

11. The fusion protein according to any one of claims 1 to 10, comprising:
- an anti-CTLA-4 antibody heavy chain fused to the IL-2 variant, wherein said heavy chain fusion has at least 85%, 90%, 95% or 98 % sequence identity with SEQ ID NO: 11 and
- an anti-CTLA-4 antibody light chain having at least 85%, 90%, 95% or 98% sequence identity with SEQ ID NO: 12.

12. A pharmaceutical composition comprising a therapeutically effective amount of the fusion protein according to any one of claims 1 to 11.

13. The pharmaceutical composition according to claim 12 for use in the treatment of cancer.

14. The pharmaceutical composition for use according to claim 13, wherein the cancer is selected from the group consisting of: Melanoma, Renal Cell Carcinoma, Colorectal Cancer, Hepatocellular Carcinoma, Non-Small Cell Lung Cancer, Malignant Pleural Mesothelioma, Esophageal Cancer, Head and Neck Squamous Cell Carcinoma, Urothelial Carcinoma, Primary Hodgkin Lynphoma, Gastric Cancer, Large B Cell Lymphoma, Cervical Cancer, Merkel Cell Carcinoma, Endometrial Carcinoma, Cutaneous Squamous Cell Carcinoma, Triple Negative Breast Cancer, Breast invasive Carcinoma, Pancreatic Adenocarcinoma, Thymoma, Prostate Adenocarcinoma, Ovarian Serous Cystadenocarcinoma, Thyroid Carcinoma, and Sarcoma.

15. The pharmaceutical composition for use according to claim 13 or 14, in combination with at least one immune check-point inhibitor; preferably anti-PD-1 and/or anti-PDL-1.
